# EUROPEAN PATENT SPECIFICATION

(11) **EP 1 663 185 B1**
(45) Date of publication and mention of the grant of the patent: **10.12.2008**
(21) Application number: 04765458.7
(22) Date of filing: 21.09.2004
(51) Int. Cl.: A61K 31/00, A61K 31/56, A61K 31/185, A61K 31/215, A61P 19/00, A61P 19/10

(54) **PREVENTION AND TREATMENT OF INFLAMMATION-INDUCED AND/OR IMMUNE-MEDIATED BONE LOSS**
PRÄVENTION UND BEHANDLUNG VON DURCH ENTZÜNDUNG AUSGELÖSTEM UND/ODER IMMUNVERMITTELTEM KNOCHENSCHWUND
PREVENTION ET TRAITEMENT DE LA PERTE OSSEUSE INDUITE PAR INFLAMMATION ET/OU IMMUNO-MEDIEE

(30) Priority: 22.09.2003 US 504717 P
(43) Date of publication of application: 07.06.2006
(73) Proprietor: onepharm Research & Development GmbH, 1210 Wien (AT)
(72) Inventor: WILCKENS, Thomas, 80796 München (DE); Dr. Ariane Volkmann, 86 911 Diessen (DE)
(74) Representative: Sonn & Partner Patentanwälte
(86) International application number: PCT/EP2004/010582
(87) International publication number: WO 2005/027882

(56) References cited:
- WO-A-02/076435
- DE-A- 2 050 072
- COOPER MARK S ET AL: "Modulation of 11beta-hydroxysteroid dehydrogenase isozymes by proinflammatory cytokines in osteoblasts: An autocrine switch from glucocorticoid inactivation to activation" JOURNAL OF BONE AND MINERAL RESEARCH, vol. 16, no. 6, June 2001 (2001-06), pages 1037-1044, XP009042532 ISSN: 0884-0431
- PATENT ABSTRACTS OF JAPAN vol. 1996, no. 03, 29 March 1996 (1996-03-29) & JP 07 291857 A (SUNTORY LTD), 7 November 1995 (1995-11-07)

## Description

The present invention relates to the use of an 11-β-HSD-type 1 and type 2 inhibitor or a pharmaceutically acceptable salt thereof for the manufacture of a pharmaceutical agent for the prevention and/or treatment of inflammation-induced and/or immune-mediated loss of bone and/or cartilage.

Morphogenesis and remodelling of bone entail the synthesis of bone matrix by osteoblasts and the coordinate resorption of bone by osteoclasts. It has been estimated that about 10% of the total bone mass in humans is being remodelled each year. Osteoblasts and osteoclasts arise from distinct cell lineages and maturation processes, that is, osteoblasts arise from mesenchymal stem cells while osteoclasts differentiate from haematopoietic monocyte/macrophage precursors.

Imbalances between osteoclast and osteoblast activities can arise from a wide variety of hormonal changes or perturbations of inflammatory and growth factors, resulting in skeletal abnormalities characterized by decreased (osteoporosis) or increased (osteopetrosis) bone mass. In fact, in pathologic states associated with inflammation, "activated" cells (e. g., infiltrating leukocytes, synovial fibroblasts, and in particular T-cells) contribute other molecules that shift the balance between osteoblastic and osteoclastic activities resulting in debilitation bone erosion and/or osteoporosis.

Increased osteoclast activity is seen in many osteopenic disorders, including postmenopausal osteoporosis, lytic bone metastases, or rheumatoid arthritis, leading to increased bone resorption and crippling bone damage. In addition, the T-cell features in diseased periodontal tissues can be compared with those in rheumatoid arthritis, wherein bone resorption often attributed to Th1-type T-cell involvement has also been demonstrated.

Various factors have been described including CSF1 (MCSF), IL1, TGFβ, TGFα, TNFα, TNFβ, IL6, vitamin 1,25-hihydroxyvitamin D3, IL11, calcitonin, PGE2, or parathyroid hormone (PTH) that affect osteoclastogenesis at distinct stages of development. However, genetic ablation experiments have shown that these factors are not essential for osteoclast development in vivo.

DE 2 050 072 A discloses to use of a pharmaceutical composition comprising 3-O-acetylglycyrrhetinic acid in the treatment of ulcer or inflammation.

Cooper et al. (Journal of Bone and Mineral Research 16 (6) 2001: 1037-1044) describe two isozymes of 11-β-hydroxysteroid-dehydrogenase (11-β-HSD) which either convert inactive cortisone to active cortisol (11-β-HSD1) or inactivate cortisol to cortisone (11-β-HSD2). Furthermore inflammatory cytokines which disable 11-β-HSD2 and induce 11-β-HSD1 are mentioned.

JP 07 291857 A discloses the use of glycyrrhetinic acid in the treatment of Paget's disease.

WO 02/076435 A describes the use of 11-β-HSD1 inhibitors for promotion of atheroprotective lipid profiles which should lead to reduced atherosclerosis and decreased rates of coronary heart disease.

US 3,934,027 discloses amide derivatives of 18-β-glycyrrhetinic acid.

Because of the enormous social and economic impacts of bone loss and crippling to human welfare and the search to increase human life span without the "side effects" of old age, it was of paramount importance to identify essential factors involved in osteoclast development and bone remodelling.
The essential molecules have been recently identified to be the TNF-TNFR superfamily proteins RANKL, RANK, and OPG. The TNF family molecule RANKL (receptor activator of NFkB ligand; also known as osteoprotegerin ligand (RANKL); TNF related activation induced cytokine (TRANCE), osteoclast differentiation factor (ODF), and TNFSF11) and its receptor RANK (TNFRSF11 A) are key regulators of bone remodelling and essential for the development and activation of osteoclasts. RANKL also regulates T-cell/dendritic cell communications, dendritic cell survival, and lymph node organogenesis.
Moreover, production of RANKL by activated T-cells directly controls osteoclastogenesis and bone remodelling and explains why autoimmune diseases, cancers, leukaemias, asthma, chronic viral infections, and periodontal disease result in systemic and local bone loss.

In particular, RANKL seems to be the pathogenetic principle that causes bone and cartilage destruction in arthritis. Inhibition of RANKL function via the natural decoy receptor osteoprotegerin (OPG, TNFRSF11B) prevents bone loss in postmenopausal osteoporosis and cancer metastases and completely blocks bone loss and crippling in various rodent models of arthritis. Intriguingly, RANKL and RANK play essential parts in the formation of a lactating mammary gland in pregnancy. This system provided a novel and unexpected molecular paradigm that links bone morphogenesis, T-cell activation and the organization of lymphoid tissues, and mammary gland formation required for the survival of mammalian species.

Inhibition of inflammation-induced and or immune-mediated osteoclast activation by blocking the activation with small molecules might be the future treatment of choice to abolish osteoporosis, tooth loss, or crippling in arthritis as well as other inflammatory process associated with bone erosion or bone loss. The latter can be achieved by preventing T-cell activation as well as bone marrow infiltration with inflammatory cells, thus inhibiting contact interaction between T-cells and osteoclast precursors, or their respective receptors and ligands RANK and RANKL.

The following section outlines the scientific rational for preventing inflammation-induced osteoclast activation in specific diseases.

### Periodontal Disease:

Host inflammatory and immune responses to specific oral bacterial infections can result in periodontal disease, i.e., periodontitis. Human periodontitis is heterogeneous in etiology, but a common halimark is alveolar bone destruction, one of the major causes of tooth loss in human. Interestingly, human periodontitis has recently been implicated in the increased risks of certain systemic disorders such as pre-term low birth weight, bacterial pneumonia, congestive heart diseases, and stroke , possibly due to an underlying inflammatory trait . About 10-12 subgingival microorganisms have been implicated in the pathogenesis of periodontitis, including *Porphyromonas gingivalis, Prevotella intermedia*, *Bacteroides forsythus,* and mixed spirochetes

In particular, *Actinobacillus actinomycetemcomitans*, a Gram-negative facultative capnophilic rod bacterium, has been identified as the etiological agent of localized juvenile periodontitis (LJP) and of some rapidly progressing and severe forms of periodontitis The prevalence of LJP is about 1-4% among teens and young adults, and 10% among insulin-dependent diabetic patients LJP is characterized by advanced alveolar bone destruction in a molar-incisor pattern that often leads to tooth mobility and loss, resulting in functional and aesthetic deficits. *A*. *actinomycetemcomitans* is able to invade the gingival epithelium and releases several virulence factors such as cytotoxins, endotoxins, and a potent leukotoxin

*A. actinomycetemcomitans* infection is usually accompanied by local and systemic antigen-specific immune responses . Earlier studies demonstrated altered CD4+/CD8+ T-cell ratios and autologous mixed lymphocyte reactions in LJP patients and the ability of T helper cells to home to periodontal tissues in rat and mouse models of periodontitis. Further, it was previously demonstrated that *A. actinomycetemcomitans* infection in NOD/SCID mice engrafted with human peripheral blood leukocytes (HuPBLs) leads to periodontal inflammation characterized by the infiltration of CD4+ T cells, CD8+ T cells, CD20+ B cells, and Maci+ macrophages into the fibrous connective tissues adjacent to the periodontal pockets .These results suggested that T cells could modulate bacterium-induced periodontal inflammation and/or alveolar bone destruction. To investigate the precise mechanism or mechanisms that regulate periodontal immunity and alveolar bone destruction, HuPBLs from LJP patients were transplanted into NOD/SCID mice (which lack endogenous T and B cells), generating HuPBL-NOD/SCID mice. This study shows that oral challenge of these "humanized" mice with *A. actinomycetemcomitans* (designated *Aa*- HuPBL-NOD/SCID) leads to functional activation of the human CD4+ T cells in the periodontium and triggers local alveolar bone destruction. *In vitro* stimulation of CD4+ T cells from these mice with antigens from *A*. *actinomycetemcomitans* leads to the expression of osteoprotegerin ligand (OPGL, also known as TRANCE, ODF, and RANKL), a key mediator of osteoclastogenesis and osteoclast activation Inhibition of OPG-L function via the decoy receptor osteoprotegerin (OPG) significantly reduces the alveolar bone destruction detected in *Aa*- HuPBL-NOD/SCID mice after bacterial inoculation, as well as the numbers of osteoclasts at the sites of local periodontal inflammation. These results identify for the first time a critical role for human CD4+ T cells reactive to oral microorganisms in periodontal disease. Moreover, *A. actinomycetemcomitans* -triggered induction of OPG-L expression on T cells and OPGL -mediated osteoclast activation and bone loss could provide one molecular explanation for the alveolar bone destruction observed in local periodontal infection.
It has recently been stated, that the concept developed above can be translated to periodontal disease in general, since the latter pathology is always accompanied by an inflammatory process resulting in T-cell activation. Periodontal disease is the second most prevalent disease in the United States after heart disease. While it affects more than 50 million people at the moderate to severe level, only 15-20% receive treatment. Currently, more than $6 billion is spent annually to treat the disease in the U.S. Periodontal disease increases the susceptibility of oral tissue and bone to degradation by bacteria, creating pockets between the teeth and gums, thus making it a major cause of tooth loss.
If left untreated, the implications of the disease extend well beyond the mouth. Studies have identified periodontal disease as a potential contributing factor to heart disease, diabetes, and low infant birth weight. The U.S. Surgeon General's Report 2000 further increased public visibility surrounding periodontal disease as a major healthcare issue. Current antimicrobial treatments cannot halt the ongoing bone destruction. Most likely a combination with small molecule preventing bone marrow infiltration with inflammatory cells and activation of T-cells will be an ideal treatment, which could be followed by a preventive strategy including the sm all molecule that blocks BM-infiltration.

### Rheumatoid arthritis:

Bone loss represents a major unsolved problem in rheumatoid arthritis (RA). The skeletal complications of RA consist of focal bone erosions and periarticular osteoporosis at sites of active inflammation, and generalized bone loss with reduced bone mass. New evidence indicates that osteoclasts are key mediators of all forms of bone loss in RA. TNF-α is one of the most potent osteoclastogenic cytokines produced in inflammation and is pivotal in the pathogenesis of RA. Production of tumor necrosis factor-α(TNF-α) and other proinflammatory cytokines in RA is largely CD4_ T-cell dependent and mostly a result of interferon-γ (IFN-γ) secretion. Synovial T cells contribute to synovitis by secreting IFN-γ and interleukin (IL)-17 as well as directly interacting with macrophages and fibroblasts through cell-to-cell contact mechanisms. Activated synovial T cells express both membrane- bound and soluble forms of receptor activator of NF-κB ligand (RANKL). In rheumatoid synovium, fibroblasts also provide an abundant source of RANKL. Furthermore, TNF-α and IL-1 target stromal-osteoblastic cells to increase IL-6, IL-11, and parathyroid hormone-related protein (PTHrP) production as well as expression of RANKL. Only in the presence of permissive levels of RANKL, TNF-α acts directly to stimulate osteoclast differentiation of macrophages and myeloid progenitor cells. In addition, TNF-α induces lL-1 release by synovial fibroblasts and macrophages, and IL-1, together with RANKL, is a major survival and activation signal for nascent osteoclasts. Consequently, TNF-α and IL-1, acting in concert with RANKL, can powerfully promote osteoclast recruitment, activation, and osteolysis in RA. The most convincing support for this hypothesis has come from *in vivo* studies of animal models. Protection of bone in the presence of continued inflammation in arthritic rats treated with osteoprotegerin (OPG) supports the concept that osteoclasts exclusively mediate bone loss, providing further evidence that OPG protects bone integrity by downregulating osteoclastogenesis and promoting osteoclast apoptosis.

The nexus between T-cell activation, TNF-α overproduction, and the RANKL/OPG/RANK ligand-receptor system points to a unifying paradigm for the entire spectrum of skeletal pathology in RA. Strategies that address osteoclastic bone resorption will represent an important new facet of therapy for RA.

### Osteoporosis in the aging population:

### A. Impact of cytokine changes with estrogen deficiency on osteoclestogenesis

There is progressive loss of bone tissue after natural or surgical menopause, leading to increased fractures within 15-20 yr from the cessation of ovarian function Estrogen receptors (ER) have been detected in many cells that reside in bone tissue suggesting that menopause may have direct consequences on cytokine secretion by cells located within the bone microenvironment. Bone marrow cells of the monocyte/macrophage lineage were believed to be the major source of the postmenopausal increases in TNF-α and IL-1 secretion in bone tissue . However, In the past few years it has been increasingly recognized that activated T cells are also an important source of increased TNF-α production in the bone marrow after menopause. Proinflammatory cytokines are among the most powerful stimulants of bone resorption known. They directly and through the stimulation of other local factors intervene with every single step in osteoclastogenesis that determines the rate of bone resorption, from the proliferation and differentiation of the early osteoclast precursor cell to the resorption capacity and the lifespan of the mature osteoclast. The first step in osteoclastogenesis that determines the rate of bone resorption is the proliferation of osteoclast precursor cells. In fact, a major consequence of estrogen deficiency is the expansion of the pool of osteoclastic precursor cells in the bone marrow. Loss of ovarian function is permissive for the expression of the major cytokines that directly stimulate early osteoclast precursor proliferation, i.e., M-CSF, GM-CSF, and IL-6. Spontaneous increases in these cytokines may be further enhanced by the parallel increases in IL-1 and TNF-α with menopause, which are potent stimulators of M-CSF, GM-CSF, and IL-6.
In summary, it can be stated that estrogen deficiency as observed after ovariectomy or in menopause is associated with an increased expression of mediatiors of inflammation. Furthermore, T-cell deficiency effectively prevented bone loss in ovariectomized mice, clearly highlighting the RANK/RANKL pathway an essential mechanism contributing to enhanced osteoclast formation and bone loss.
Of note, estrogen deficiency also appears to correlate with the incidence of several autoimmue diseases linking T-cell, B-cell activation with hormone status and bone physiology.
As outlined above, bone loss with estrogen deficiency involves a large number of interrelated changes in estrogen-dependent regulatory factors. However, whereas in other proinflammatory conditions such as inflammatory arthritis, the deficiency in single proinflammatory cytokines does not fully prevent the inflammatory process, deficiency in several single cytokines is sufficient to completely block excessive bone resorption with estrogen deficiency. The redundancy of the function of most of these cytokines for osteoclast formation may compensate the lack of function of each of these components in situations apart from estrogen deficiency. The clear exceptions are M-CSF and the components of the RANKL, OPG/RANKsystem, whose activity is essential for osteoclast generation. This evidence makes blockade of the T-cell interaction with osteoclast precursors a most attractive avenue for new therapeutic intervention in estrogen-induced bone loss; the latter being consider similar to inflammation-induced bone destruction.

The cortisone/cortisol shuttle:
The interconversion of pharmacologically active cortisol and inactive cortisone is accomplished by two independent 11-β-hydroxysteroid dehydrogenases (11-β-HSD)3 that exhibit tissue-specific expression. Even though a third enzyme has been proposed, its existence has still to be demonstrated. In most intact cells, 11 β-HSD1 functions predominantly as a reductase, generating active cortisol from inactive cortisone and thereby enhancing activation of the glucocorticoid receptor. However, there is strong evidence, that the reaction direction might highly depend on the specific tissue type; thus in Leydig cells 11-β-HSD-1 may also function as a dehydrogenase. 11-β -HSD1 is broadly distributed among tissues, with predominant expression occurring in hepatic, adipose, gonadal, and central nervous system tissues. Mice with a targeted disruption of the 11-β-HSD1 gene are more resistant to hyperglycemia induced by stress or high-fat diet than their wildtype counterparts, consistent with the emerging notion that the activation of glucocorticoids by prereceptor metabolism may be central to the appearance of many sequelae of insulin resistance 11-β-HSD2, which is mainly expressed in the placenta and aldosterone target tissues such as the kidney and colon, acts almost exclusively as a dehydrogenase, thereby preventing the activation of mineralocorticoid receptor-sensitive genes by excess cortisol.

18-β-Glycyrrhetinic acid, an active component of licorice is an inhibitor of 11-β-HSD1 as well as 11-β-HSD2, and licorice ingestion or administration of 18 β-glycyrrhetinic acid or its hemisuccinate derivative carbenoxolone results in hypertension and metabolic alkalosis due to inhibition of 11-β-HSD2 due to increased access to active cortisol to the mineralocorticoid receptors in the kidney. Patients with mutations in the gene encoding 11-βP-HSD2 suffer from the syndrome of "apparent mineralocorticoid excess" entailing hypokalemia and severe hypertension . Similar symptoms also were recently described for the 11-β-HSD2 knockout mice.
For several decades, synthetic glucocorticoids have found significant therapeutic use as anti-inflammatory agents in various diseases such as rheumatoid arthritis, allergic diseases, and bronchial asthma . Consistent with the pluripotent effects of glucocorticoids, the glucocorticoid receptor is widely distributed among peripheral tissues. In many instances, the tissue distribution of this receptor and that of 11-β-HSD1 are overlapping. Although glucocorticoids are commonly prescribed for their anti-inflammatory actions, to date relatively few studies address the involvement of 11-β-HSD in glucocorticoid-mediated immune functions. In one such study, the importance of pre-receptor metabolism by 11β-HSD enzymes in controlling inflammatory responses has been highlighted by demonstrating that pharmacological inhibition of 11β-HSD activity present in skin leads to an augmentation of the anti-inflammatory action of topically applied cortisol on contact hypersensitivity responses .The inhibitor applied alone displayed no effect. There it was proposed that blocking 11-β-HSD in the skin abrogated corticoid inactivation.
Recently the expression of 11-β-HSD in a primary inflammatory effector cell, the monocyte/macrophage was investigated. These studies confirm the complete absence of both 11β-HSD1 and 11β- HSD2 in freshly Isolated circulating human monocytes. However, 11β-reductase activity was induced during monocyte culture or after stimulation with the anti-inflammatory cytokines IL-4 and IL-13, strongly suggesting that it may play an important role in regulating the immune functions of these cells.

Since both isoenzymes were discovered in bone cells, it was further speculated that activation of cortisone by the dominant reductase activity of 11-β-HSD, e.g. exaggerated conversion to cortisol might be part of bone loss induced by glucocorticoids in general, including osteoporosis observed in rheumatoid arthritis. From this evidence one could speculate that blocking 11-β-HSD would result in enhanced bone loss.

Thus, while we had proposed that blocking 11-β-HSD would not only ameliorate arthritis by enabling tolerance induction due to increased local glucocorticoid concentrations, we were concerned that this treatment would increase bone destruction.

Surprisingly this is not the case. In fact, blockade of 11-β-HSD not only decreased inflammation, but also completely prevented bone marrow infiltration with inflammatory cells. Since it has been proposed that preosteoclasts are recruited from synovial as well as bone marrow monocytic cell lines, the prevention of infiltration must be considered the main effector pathway for the prevention of bone erosion in adjuvant arthritis and inflammation-induced bone destruction in general. The latter is further corroborated by the fact that the injection of 18-β-glycyrrhetinic acid needed to be in close proximity to draining lymph nodes in order to display clinical efficacy either alone or in conjunction with a peptide.

Therefore we propose that 11-β-HSD blockade increases local glucocorticoid concentrations in immune tissues which prevents the interaction between activated T-cells an osteoclast precursors and/or T-cell activation *per se*.

Given these findings it appears most unlikely that endogeous glucocorticoids contribute to bone loss during acute inflammation; the latter might possibly be the case under physiological non-inflammatory conditions. In fact, in rat adjuvant arthritis, an established model for the human disease, dexamethasone, a potent synthetic glucocorticoid in conjunction with a CD4+ depleting antibody, strongly protected rats from bone erosion- In addition, dexamethasone also enhanced anti-TNF-induced amelioration of synovial inflammation and bone erosion in rat models for rheumatoid arthritis. Thus increasing local glucocorticoid levels might have benefical effects on bone and bone homeostasis during acute inflammation and/or during immune-mediated activation of bone destruction. Our findings clearly contraste the hypothesis recently put forward.
In addition, 11-β-HSD expressed in osteoblasts is most unlikely to play a role in the present phenomenon, since activation of osteoclast is depending on the interaction with activated T-cells, and not osteoblast in bone marrow. This evidence further negates a functional role of osteoblastic 11-β-HSD in inflammation induced bone distruction.
Based on our *in vivo* findings we investigated the gene expression of 11-β-HSD and biological activity in tissues relevant for immune function. For the first time we identified 11-β-HSD activity in dendritic cells and lymphoid cells (unpublished) in both, human and rat tissues. Most interestingly, taqman analysis indicates the presence of mRNA for more then one 11-β-HSDs. This evidence strongly suggest that 11-β-HSD might have a functional role in regulating immunity. In addition, the previously postulated type 3 enzyme might well be a homologue of the established type 2. It had earlier been proposed that differences might potentially exist within the known 11-β-HSD-2 enzymes observed in placenta and kidney, since their cDNAs were similar but not identical.
Since 18-β-glycyrrhetinic acid blocks both known as well as a putative third enzyme, it currently can not be definitely decided which enzyme is the most responsible for the beneficial effect of 11-β-HSD-blockade. The fact that inflammatory mediators such as cytokines can influence the balance between reductase and dehydrogenase activity either by altering the balance between the iso-enzymes or changing the reaction direction at the single enzyme level₅ necessitates the development of more selective inhibitors for identification of the relevant target.

Recent evidence establishes inflammation-induced and/or immune-mediated bone loss as an essential direct interaction between activated T-cells and osteoclast precursors. This crucial mechanism can be prevented by the use of 18-β-glycyrrhetinic acid and related compounds that modulate the cortisol/cortisone shuttle; i.e. 11-β-hydroxysteroid-dehydrogenase activity and/or expression as well as selective inhibitors useful for the modulation of 11-β-HSD.

It was an object of the present invention to provide a pharmaceutical agent for the prevention and/or treatment of inflammation-induced and/or immune-mediated loss of bone and/or cartilage.

Therefore the present invention relates to a use of an 11-β-HSD-type 1 and type 2 inhibitor or a pharmaceutically acceptable salt thereof, for the manufacture of a pharmaceutical agent for the prevention and/or treatment of inflammation-induced and/or immune-mediated loss of bone and/or cartilage, wherein said use is for the prevention and/or treatment of lytic bone metastases, arthritis, juvenile chronic arthritis and/or adjuvant arthritis, infectious diseases, bone loss by HIV, tooth loss, bone marrow inflammation and/or synovial inflammation.

Using conventional drugs for the therapy of inflammations, it was observed that bone loss continues to go on, since osteoclast activation remains. It was found that bone loss can be prevented effectively by means of the 11-β-HSD inhbitors of the invention.

According to the invention, the 11-β-HSD-type 1 and type 2 inhibitors are preferably used for the prevention and/or treatment of bone and/or cartilage loss in a mammal, more preferably in a human.

It is contemplated that an inflammation-induced and/or immune-mediated loss of bone and/or cartilage may include osteoporosis, postmenopausal osteoporosis, lytic bone metastases, arthritis, juvenile chronic arthritis, adjuvant arthritis, infectious diseases, bone loss by cancer, bone loss by HIV, tooth loss, bone marrow inflammation, synovial inflammation, cartilage and/or bone erosion and/or proteoglycan damage.

In a more preferred embodiment of the invention, the immune-mediated loss of bone and/or cartilage includes osteoarthritis, rheumatoid arthritis and/or periodontitis.

Preferably, the 11-β-HSD-type 1 and type 2 inhibitors are selected from the group consisting of the following formulas:

| **Compound Name** | **Structure** |
|---|---|
| Formula 1 | 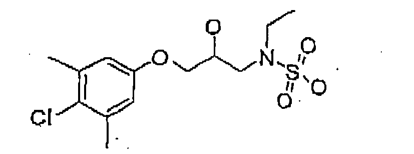 |
| Formula 2 | 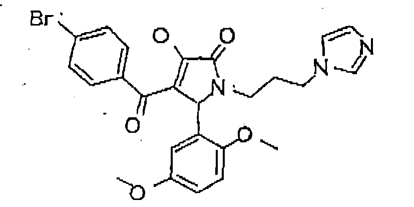 |
| Formula 3 | 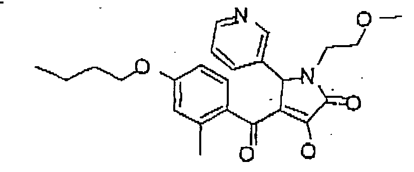 |
| Formula 4 | 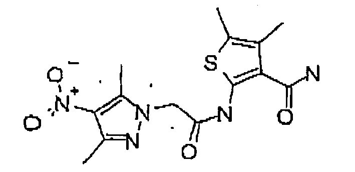 |
| Formula 5 | 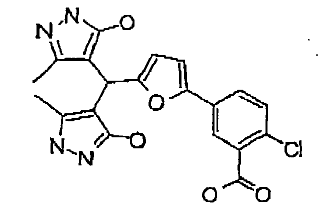 |
| Formula 6 | 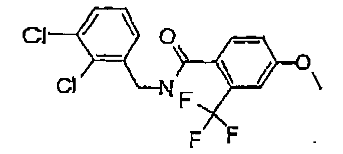 |
| Formula 7 | 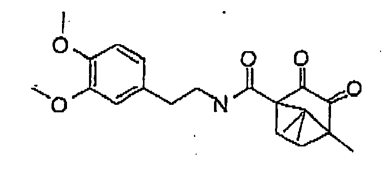 |
| Formula 8 | 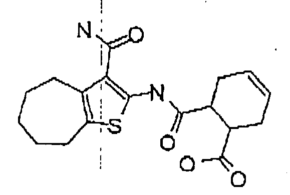 |
| Formula 9 | 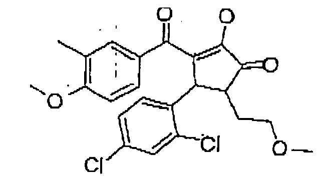 |
| Formula 10 | 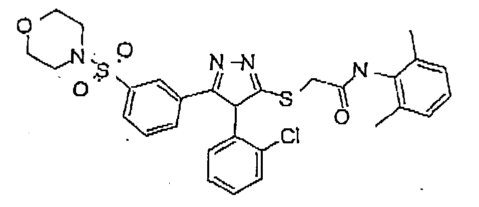 |
| Formula 11 | 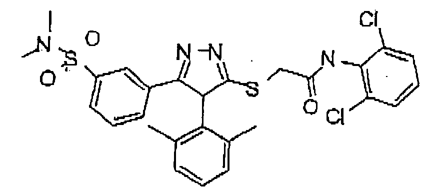 |
| Formula 12 | 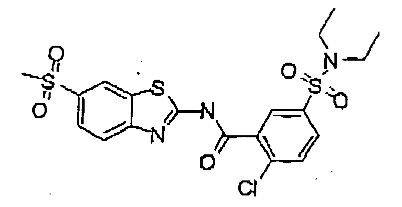 |
| Formula 13 | 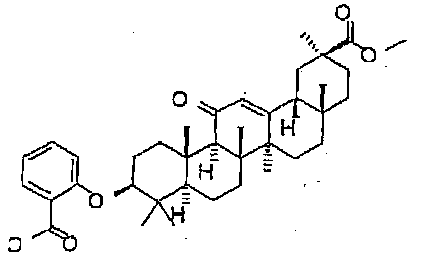 |
| Formula 15 | 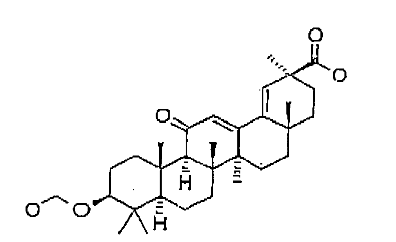 |
| Formula 16 | 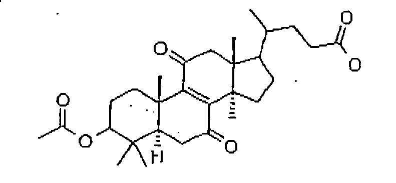 |
| Formula 17 | 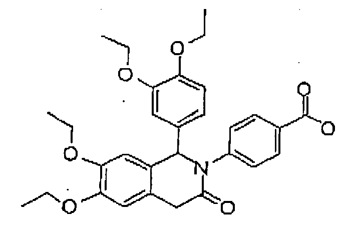 |
| Formula 18 | 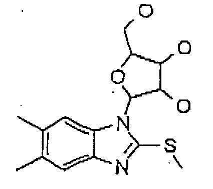 |
| Formula 19 | 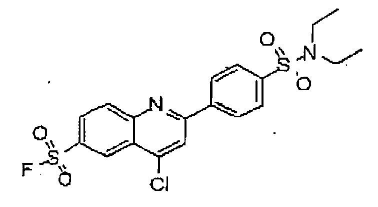 |
| Formula 20 | 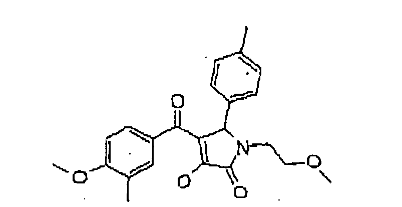 |
| Formula 21 | 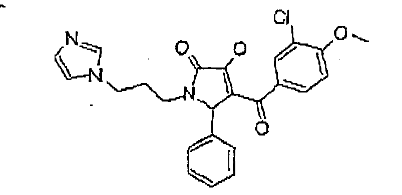 |
| Formula 22 | 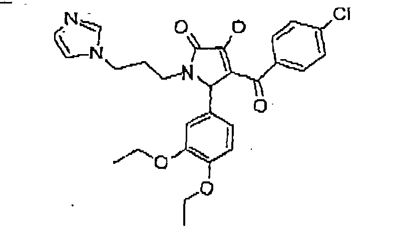 |
| Formula 23 | 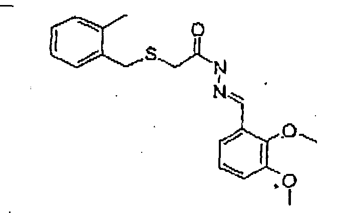 |
| Formula 25 | 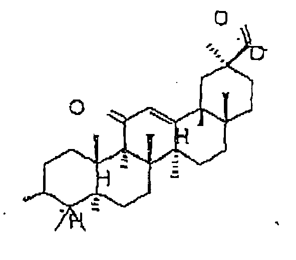 |
| Formula 26 | 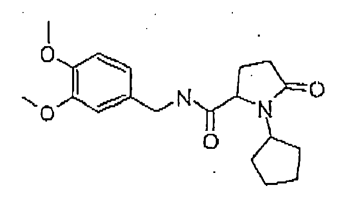 |
| Formula 27 | 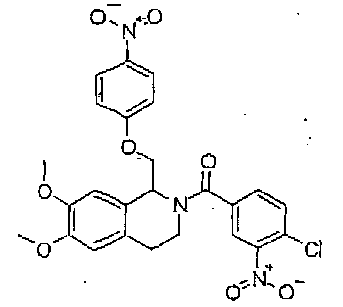 |
| Formula 28 | 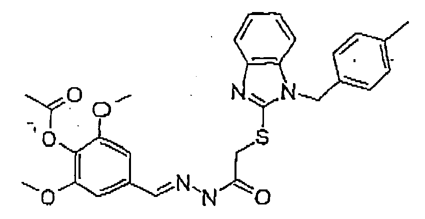 |
| Formula 29 | 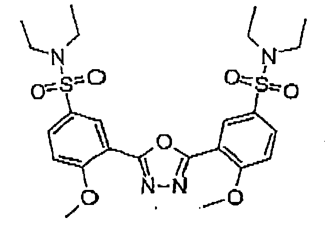 |
| Formula 30 | 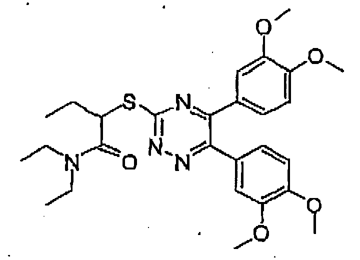 |
| Formula 31 | 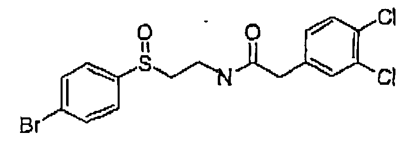 |

In a preferred embodiment of the invention, the 11-β-HSD-type 1 and type 2 inhibitors are selected from the group consisting of the formulas 13 and 25 as follows;

| | |
|---|---|
| Formula 13 | 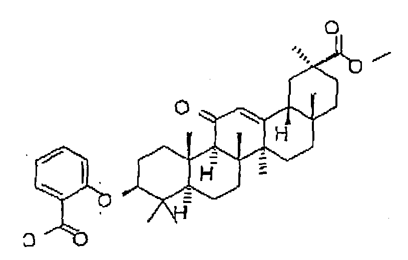 |
| Formula 25 | 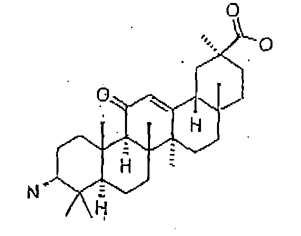 |

Said structures were found to be particularly effective in the specific inhibition of 11-β-HSD, preferably of 11-β-HSD-1, 11-β-HSD-2 and/or 11-β-HSD-1 and 2.

Preferably, the inhibitor is of formula 16:

| | |
|---|---|
| Formula 16 | 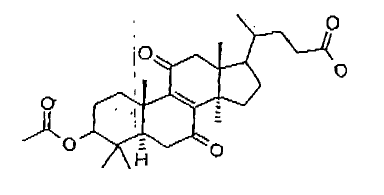 |

In a further preferred embodiment of the invention, the 11-β-HSD-type 1 and type 2 inhibitor is formula 7:

| | |
|---|---|
| Formula 7 | 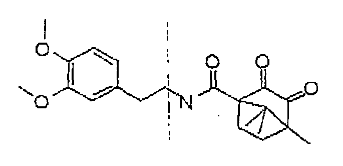 |

Further contemplated 11-β-HSD-1 or -2 inhibitors are for the prevention and/or treatment of inflammation- induced and/or immune-mediated bone loss, for example, but not limited to, 18-β-glycyrrhetinicacid, progesterone, 5α-dihydroprogesterone, 5β-dihydroprogesterone, 20α-dihydroprogesterone; 3β5α-tetrahydroprogesterone, 17α-OH-progesterone, 20α-dihydro-5α-dihydroprogesterone, 20α-Dihydroprogesterone, 11α-OH-progesterone, 11β-OH-progesterone, corticosterone, 11β-OH-androstenoidone, 3-alpha, 5-beta-tetrahydroprogesterone, 3-alpha, 5-beta-tetrahydro-11-deoxy-corticosterone, 11-epicortisol, chenodeoxycholic acid, cholic acid, glycyrrhetinic acid (3β-hydroxy-11-oxooleane-12-ene-30-acid) and derivatives thereof such as glycyrrhicine, glycyrrhicinic acid and carbenoxolone; furosemide and derivatives thereof, flavonoides and derivatives thereof such as naringenine, triterpinoides (e.g. CHAPS), ketokonazole, saiboku-to, gossypol, metyrapone, 11-epiprednisolone. Further suitable inhibitors are steroid-like, such as dexamethasone, budesonide, deflazacort and stanozolol.

The 11-β-HSD-type 1 and type 2 inhibitors of the present invention can be utilized in the prevention and/or treatment of inflammation-induced and/or immune-mediated loss of bone and/or cartilage alone or in combination with at least one active ingredient being effective in the prevention and/or treatment of inflammation-induced and/or immune-mediated loss of bone and/or cartilage.

The drug products are produced by using an effective dose of the compounds of the invention or salts thereof, in addition to conventional adjuvants, carriers and additives. The dosage of the pharmaceutical agents may vary depending on the mode of administration, the age and weight of the patient, the nature and severity of the disorders to be treated and similar factors. The daily dose may be given as a single dose to be administered once a day, or divided into two or more daily doses, and is usually 5-100 mg/kg body weight, preferably 7-80 mg/kg body weight, more preferably 10-50 mg/kg body weight and most preferred 20 mg/kg body weight, related to a person weighing 70 kg.

Oral, sublingual, intravenous, intramuscular, intraarticular, intraarterial, intramedullar, intrathecal, intraventricular, intraocular, intracerebral, intracranial, respiratoral, intratracheal, nasopharhyngeal, transdermal, intradermal, subcutaneous, intraperitoneal, intranasal, enteral and/or topical administration and/or administration via rectal means, via infusion and/or via implant are suitable according to the invention. Oral administration of the compounds of the invention is particularly preferred. Galenical pharmaceutical presentations such as tablets, coated tablets, capsules, dispersible powders, granules, aqueous solutions, aqueous or oily substances, sirup, solutions or drops are used.

Solid drug forms may comprise inert ingredients and carriers such as, for example, calcium carbonate, calcium phosphate, sodium phosphate, lactose, starch, mannitol, alginates, gelatin, guar gum, magnesium stearate or aluminium stearate, methylcellulose, talc, colloidal silicas, silicone oil, high molecular weight fatty acids (such as stearic acid), agar-agar or vegetable or animal fats and oils, solid high molecular weight polymers (such as polyethylene glycol); preparations suitable for oral administration may, if desired, comprise additional flavourings and/or sweetners.

Liquid drug forms can be sterilized and/or, where appropriate, can comprise excipients such as preservatives, stabilizers, wetting agents, penetrants, emulsifiers, spreading agents, solubilizers, salts, sugars or sugar alcohols to control the osmotic pressure or for buffering and/or viscosity regulators.

Examples of such additions are tartrate buffer and citrate buffer, ethanol, complexing agents (such as ethylenediaminetetraacetic acid and its non-toxic salts). Suitable for controling the viscosity are high molecular weight polymers such as, for example, liquid polyethylene oxide, microcrystalline celluloses, carboxymethylcelluloses, polyvinylpyrrolidones, dextrans or gelatin. Examples of solid carriers are starch, lactose, mannitol, methylcellulose, talc, colloidal silicas, higher molecular weight fatty acids (such as stearic acid), gelatin, agar-agar, calcium phosphate, magnesium stearate, animal and vegetable fats, solid high molecular weight polymers such as polyethylene glycol.

Oily suspensions for parenteral or topical uses may be vegetable, synthetic or semisynthetic oils such as, for example, liquid fatty acid esters with, in each case, 8 to 22 C atoms in the fatty acid chains, for example palmitic, lauric, tridecyclic, margaric, stearic, arachic, myristic, behenic, pentadecyclic, linoleic, elaidic, brasidic, erucic or oleic acid, which are esterified with monohydric to trihydric alcohols having 1 to 6 C atoms, such as, for example, methanol, ethanol, propanol, butanol, pentanol or iosmers thereof, glycol or glycerol. Examples of such fatty acid esters are commercially available miglyols, isopropyl myristate, isopropyl palmitate, isopropyl stearate, PEG 6-capric acid, caprylic/capric esters of saturated fatty alcohols, polyoxyethylene glycerol trioleates, ethyl oleate, waxy fatty acid esters such as artificial duch preen gland fat, coco fatty acid, isopropyl ester, oleyl oleate, decyl oleate, ethyl lactate, dibutyl phthalate, diisopropyl adipate, polyol fatty acid esters inter alia. Also suitable are silicone oils differing in viscosity or fatty alcohols such as isotridecyl alcohol, 2-octyldodecanol, cetylstearyl alcohol or oleyl alcohol, fatty acids such as, for example, oleic acid. It is also possible to use vegetable oils such as caster oil, almond oil, olive oil, sesame oil, cottonseed oil, peanut oil or soybean oil.

Suitable solvents, gel formers and solubilizers are water or water-miscible solvents. Suitable examples are alcohols such as, for example, ethanol or isopropyl alcohol, benzyl alcohol, 2-octyldodecanol, polyethylene glycols, phthalates, adipates, propylene gylcol, glycerol, di- or tripropylene gylcol, waxes, methyl Cellosolve, Cellosolve, esters, morpholines, dioxane, dimethyl sulfoxide, dimethylformamide, tetrahydrofuran, cyclohexanine, etc.

Film formers which can be used are cellulose ethers able to dissolve or swell both in water and in organic solvents such as, for example, hydroxypropylmethylcellulose, methylcellulose, ethylcellulose or soluble starches.

Combined forms of gel formers and film formers are also possible. In particular, ionic macromoelcules are used for this purpose, such as, for example, sodium carboxymethylcellulose, polyacrylic acid, polymethylacrylic acid and salts thereof, sodium amylopectin semiglycolate, alginic acid or propylene glycol alginate as sodium salt, gum arabic, xanthan gum, guar gum or carrageenan.

Further formulation aids which can be employed are glycerol, paraffin of differing viscosity, triethanolamine, collagen, allantoin, novantisolic acid.

It may also be necessary to use surfactants, emulsifiers or wetting agents for the formulation, such as, for example, Na lauryl sulfate, fatty alcohol ether sulfates, di-Na-N-lauryl-β-iminodipropionate, polyethoxylated castor oil or sorbitan monooelate, sorbitan monostearate, polysorbates (e.g. Tween), cetyl alcohol, lecithin, glyceryl monostearate, polyoxyethylene stearate, alkylphenol polyglycol ether, cetyltrimethylammonium chloride or mono/dialkylpolyglycol ether orthophosphoric acid monoethanolamine salts. Stabilizers such as montmorillonites or colloidal silicas to stabilize emulsions or to prevent degradation of the active substances, such as antioxidants, for example tocopherals or butylated hydroxyanisole, or preservatives such as p-hydroxybenzoic esters, may likewise be necessary where appropriate to prepare the desired formulations.

Preparations for parenteral administration may be present in separate dose unit forms such as, for example, ampoules or vials. Solutions of the active ingredient are preferably used, preferably aqueous solutions and especially isotonic solutions, but also suspensions. These injection forms can be made available as a finished product or be prepared only immediately before use by mixing the active compound, e.g. the lyophilistate, where appropriate with further solid carriers, with the desired solvent or suspending agent.

Intranasal preparations may be in the form of aqueous or oily solutions or of aqueous or oily suspensions. They may also be in the form of lyophilistates which are prepared before use with the suitable solvent or suspending agent.

The manufacture, bottling and closure of the products takes place under the usual antimicrobial and aseptic conditions.

In another preferred embodiment of the Invention, the pharmaceutical composition is selected from the group consisting of the formula 13 and 25 as defined above.

More preferably, the structure is formula 16 as defined above.

In another embodiment of the present invention, the pharmaceutical composition has formula 7 as defined above.

According to the invention, a pharmaceutical composition is preferably for the prevention and/or treatment of inflammation-induced and/or immune-mediated loss of bone and/or cartilage, more preferably for the prevention and/or treatment of osteoporosis, postmenopausal osteoporosis lytic bone metastases, arthritis, osteoarthritis, rheumatoid arthritis, juvenile chronic arthritis, chronic arthritis, adjuvant arthritis, infectious diseases, bone loss by cancer, bone loss by HIV, periodontitis, bone marrow inflammation, synovial inflammation, cartilage/bone erosion and/or proteoglycan damage.

The pharmaceutical composition of the present invention, in addition to an 11-β-HSD-type 1 and/or type 2 inhibitor and a pharmaceutically acceptable carrier or diluent, can comprise at least one active ingredient being effective in the prevention and/or treatment of inflammation-induced and/or immune-mediated loss of bone and/or cartilage.

The pharmaceutical compositions may be administered by any number of routes including, but not limited to oral, sublingual, intravenous, intramuscular, intraarticular, intraarterial, intramedullar, intrathecal, intraventricular, intraocular, intracerebral, intracranial, respiratoral, intratracheal, nasopharhyngeal, transdermal, intradermal, subcutaneous, intraperitoneal, intranasal, enteral and/or topical and/or via rectal means, via infusion and/or implant. Preferably, said route of administration is oral.

The term "pharmaceutically acceptable" means a non-toxid material that does not interfere with the effectiveness of the biological activity of the active ingredients. Such preparations may routinely contain pharmaceutically acceptable concentrations of salts, buffering agents, preservatives, compatible carriers, supplementary immune potentiating agents such as adjuvants and cytokines and optionally other therapeutic agents such as chemotherapeutic agents.

When used in medicine, the salts should be pharmaceutically accceptable, but non-pharmaceutically acceptable salts may conveniently be used to prepare pharmaceutically acceptable salts thereof and are not excluded from the scope of the invention.

The pharmaceutical compositions may contain suitable buffering agents, including acetic acid in a salt; citric acid in a salt; boric acid in a salt; and phosphoric acid in a salt.

The pharmaceutical compositons optionally may also contain suitable preservatives such as benzalkonium chloride, chlorobutanol, parabenes and thiomersal.

The pharmaceutical compositions may conveniently be presented in unit dosage form and may be prepared by any of the methods well-known in the art of pharmacy. All methods include the step of bringing the active agent into association with a carrier which constitutes one or more accessory ingredients. In general, the compositions are prepared by uniformly and intimately bringing the active compound into association with a liquid carrier, a finely divided solid carrier or both, and then, if necessary, shaping the product.

Compositions suitable for oral administration may be presented as discrete units such as capsules, tablets, lozenges, each containing a predetermined amount of the active compound. Other compounds include suspensions in aqueous liquids or non-aqueous liquids such as sirup, elixir or an emulsion.

Compositions suitable for parenteral administration conveniently comprise a sterile aqueous or non-aqueous preparation which is preferably isotonic with the blood of the recipient. This preparation may be formulated according to known methods using suitable dispersing or wetting agents and suspending agents. The sterile injectable preparation also may be a sterile injectable solution or suspension in a non-toxic parenterally acceptable diluent or solvent, for example, as a solution in 1,3-butane diol. Among the acceptable vehicles and solvents that may be employed are water, Ringer's solution, and isotonic sodium chloride solution. In addition, sterile fixed oils are conventionally employed as a solvent or suspending medium. For this purpose, any bland fixed oil may be employed including synthetic mono- or diglycerides. In addition, fatty acids such as oleic acid may be used in the preparation of injectables.

Carrier formulations suitable for oral, subcutaneous, intravenous, intramuscular etc. administrations can be found in Remington's Pharmaceutical Sciences, Mack Publishing Co., Easton, PA.

In a preferred embodiment of the invention, the pharmaceutical compositions are administered to a mammal, preferably a human, in a dose of 5-100 mg/kg body weight per day, more preferably 7-80 mg/kg body weight per day, still more preferably 10-50 mg/kg body weight per day and most preferably 20 mg/kg body weight per day. This dose refers to a person weighing 70 kg.

In another preferred embodiment of the invention, the pharmaceutical composition is for the inhibition of osteoclast activity, since imbalances between osteclast and osteoblast activities toward the osteclast activities results in skeletal abnormalities characterized by loss of bone and/or cartilage.

### Examples

### Example 1

### Adjuvant-induced arthritis (AIA)

An intradermal injection, at the base of the tail, with heat killed Mycobaterium tuberculosum in incomplete Feund's Adjuvans results in destructive arthritis within 14 days in susceptible DA or LEW inbred rat strains. AIA can also be induced with cell walls from other bacterial types in IFA, although the arthritogenicity varies. Increased synthesis of tumor necrosis factor a (TNF-a), interleukin 1 (IL-1) and IL-6 is detected as early as day four after adjuvant injection. The disease progresses rapidly over several weeks in what appears clinically to be a monophasic process.
Granulocytes and autoreactive CD41 cells play major roles in the disease. Humoral immune mechanisms appear not to contribute to the disease process. This unique rat disease model represents a systemic process that involves not only the joints but also the gastrointestinal and genitourinary tracts, the skin and the eyes. Although AIA clinically and histologically has similarities to human rheumatoid arthritis.

In this animal model it has impressively been demonstrated that bone loss and partially the related cartilage destruction essentially depends on the activation of osteoclasts by T-cells.
Therefore this animal model ideally serves to investigate mechanisms and targets that might be suitable for the development of novel therapeutics with improved therapeutic efficacy. In fact, most current treatments for arthritis and other conditions associated with immune mediated bone loss only ameliorate inflammation but fail to halt bone and cartilage loss.

Figure 2 shows the effect of 18-β-glycyrrhetinic acid (BX-1) on inflammation, as well as bone and cartilage loss.
BX-1 early: BX-1 injected i.d. at the time of disease induction (day0) and day2, day4
BX-1 late: BX-1 injected i.d. at first signs of arthritis, day9, day11, day13
Samples are from both left and right hind limb of three rats per group of a representative experiment

### Data are shown as SEM

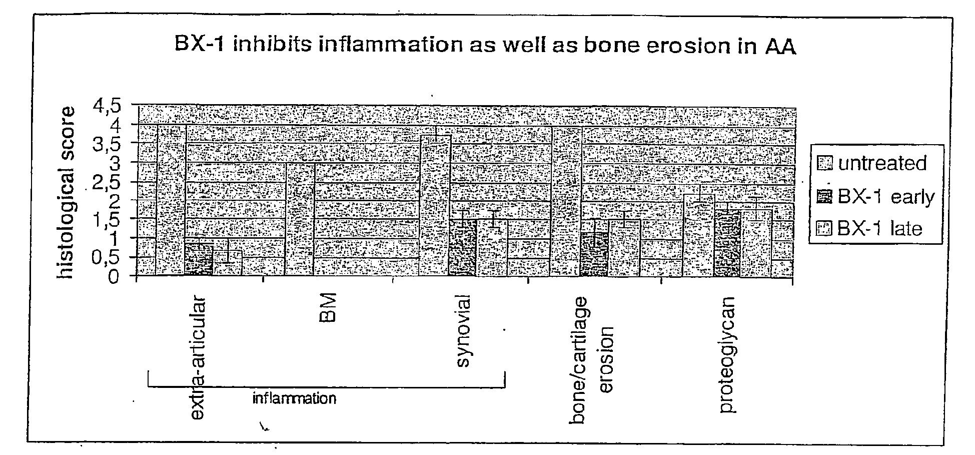

### Histology

Excised rat joints were stained with H&E. A synovial histology score was determined on the stained sections using a semiquantitative scale that measures synovial inflammation (0-4), bone and cartilage erosions (0-4), marrow infiltration (0-4), and extra-articular inflammation (0-4) (maximum score, 16).

### Statistics

Two-tailed unpaired Student *t* tests were used to compare Ab levels, cytokine levels, clinical arthritis scores, and histology scores using StatView (SAS Institute, Cary, NC) and Mathsoft computer software (Mathsoft, Cambridge, MA).

### Histological results of hind joint sections from arthritic rats

Rat ankle slides were histologically evaluated according to five criteria (blind evaluation by DL Boyle et al., University of California in San Diego, (J. Immunol., Jan 2002; 168: 51 - 56.):
1. Extra-articular inflammation
2. Bone marrow inflammation (BM)
3. Synovial inflammation
4. Cartilage/bone erosion
5. Proteoglycan damage

The complete lack of infiltration of the bone marrow has not been observed with any short term and/or discontinued treatment with a small molecule drug before.

The data further indicate that BX-1 (18-β-glycyrrhetinic acid) positively influence all arms of the pathology of arthritis; T-cell and dendritic cell activation, systemic inflammation, and bone marrow infiltration. Similar effects were seen with the hemisuccinate of BX-1, carbenoxolone (not shown).

The histological findings might explain why the animals go in remission upon late treatment, i.e. after the onset of disease and why there is absolutely no sign of re-exacerbation of disease after cessation of treatment in any model we have investigated so far; i.e. adjuvant arthritis and pristane-induced arthritis (not shown),

Over all, these data suggest that BX-1 may be an ideal drug to reduce inflammation-induced and/or immune bone destruction as observed not only in rheumatoid arthritis, but also periodontal diseases and other inflammatory conditions. In fact, the pathology of periodontal diesase and other pathologies resulting in bone destruction appears to follow a similar pathway as this is currently accepted for bone destruction in rheumatoid arthritis (Annu. Rev. Immunol., Jan 2002; 20: 795 - 823), which opens new, *ad hoc* opportunities for BX-1 and related drugs. Since BX-1 is an established inhibitor of 11-β-HSD type 1 and type 2, enzymes blocking these with inhibitors appears a most promising avenue to cure diseases associated with inflammation and/or immune mediated bone loss.

### Example 2

### Materials

Cell culture reagents were purchased from Invitrogen (Carlsbad, CA), [1,2,6,7-³H]-cortisone from American Radiolabeled Chemicals (St. Louis, MO) and [1,2,6,7-³H]-cortisol from Amersham Biosciences (General Electrics Healthcare, Piscataway, NJ). Thin layer chromatography (TLC) plates (SIL G-25 UV254) were purchased from Macherey-Nagel, Oensingen, Switzerland.

### Assay for 11β-HSD activity

The screening assay used to determine inhibition of 11β-HSD enzyme activity is based on the conversion of radiolabelled cortisone or cortisol in cell lysates from HEK-293 cells, stably transfected with either human 11β-HSD1 or human 11β-HSD2 (Schweizer et al. 2003, Frick et a. 2004). Cells were grown in 10 cm dishes to 80% confluence and incubated for 16 h in steroid-free medium (charcoal-treated fetal calf serum (FCS) from HyClone, Logan, Utah). Cells were rinsed once with phosphate-buffered saline (PBS), dettached and centrifuged for 3 min at 150 x g. The supernatant was removed and the cell pellet quick-frozen in a dry-ice ethanol bath. At the day of experiment, cell pellets were resuspended in buffer TS2 (100 mM NaCl, 1 mM EGTA, 1 mM EDTA, 1 mM MgCl₂, 250 mM sucrose, 20 mM Tris-HCl, pH 7.4), sonicated and activities determined immediately. The rate of conversion of cortisol to cortisone or the reverse reaction was determined in 96-well optical PCR reaction plates (Applied Biosystems, Foster City, CA) in a final volume of 22 µl, and the tubes were capped during the reaction to avoid evaporation.

### Determination of oxidase activity:

Reactions were initiated by simultaneously adding 10 µl of cell lysate and 12 µl of TS2 buffer containing the appropriate concentration of the inhibitory compound to be tested, NAD⁺, 30 nCi of [1,2,6,7-³H]-cortisol and unlabeled cortisol. A final concentration of 400 µM NAD⁺ and 25 nM cortisol were used. Stock solutions of the inhibitors in methanol or DMSO were diluted in TS2 buffer to yield the appropriate concentrations, whereby the concentration of methanol or DMSO in the reactions were kept below 0.1%. Control reactions with or without 0.1% of the solvent were performed. Incubation was at 37°C for 10 min with shaking, reactions were terminated by adding 10 µl of stop solution containing 2 mM of unlabeled cortisol and cortisone dissolved in methanol. The conversion of radiolabeled cortisol was determined by separation of cortisol and cortisone using TLC and a solvent system of 9:1 (v/v) chloroform:methanol, followed by scintillation counting. In absence of inhibitors approximately 30% of cortisol was converted to cortisone.

### Determination of reductase activity:

Reactions were initiated simultaneously by adding 10 µl of cell lysate and 12 µl of TS2 buffer containing the appropriate concentration of the inhibitory compound to be tested, NADPH, 30 nCi of [1,2,6,7-³H]-cortisone and unlabeled cortisone, whereby final concentrations were 400 µM NADPH and 100 nM cortisone. Activities were determined immediately after cell disruption by measuring the conversion of radiolabeled cortisone to cortisol for 10 min.

Enzyme kinetics were analyzed by non-linear regression using Data Analysis Toolbox (MDL Information Systems Inc.) assuming first-order rate kinetics. Data represent mean ± SD of four to five independent experiments.

### References

Schweizer, R A., Atanasov, A. G., Frey, B. M., and Odermatt, A. (2003) Mol Cell Endocrinol 212, 41-49.
Christoph Frick, Atanas G. Atanasov, Peter Arnold, Juris Ozols, and Alex Odermatt (2004) J Biol Chem, 279, 131-138.

### Example 3

Inhibition of 11β-HSD1 was determined at 100 nM cortisone, inhibtion of 11β-HSD2 at 25 nM cortisol as substrates (at approximately 30% of apparent Km concentrations).

Assay with 20 µM of the corresponding compound in the reaction mixture, added simultaneously with the substrate:

| 11β-HSD1 | 11β-HSD1 | % of control | 11β-HSD2 | % of control |
|---|---|---|---|---|
| control | | 99.9999986 | | 100 |
| 10 µM CBX | | 4.43030125 | | 15.52151455 |
| BNW 1 | | 102.112595 | | 96.77455646 |
| BNW2 | | 78.8440316 | | 77.95067459 |
| BNW3 | | 60.2536577 | | 53.56660046 |
| BNW4 | | 82,2425505 | | 95.04764105 |
| BNW5 | | 69.7522595 | | 97.47129918 |
| BNW6 | | 79.6439869 | | 145.0319346 |
| BNW7 | | 9.59257261 * | | 139.5062669 |
| BNW8 | | 41.7056688 | | 102.7042587 |
| BNW9 | | 30.6544131 | | 77.43471825 |
| BNW10 | | 64.325535 | | 128.6701314 |
| BNW11 | | 70.0994104 | | 120.918247 |
| BNW12 | | 85.3624514 | | 132.1217751 |
| BNW13 | | 3.87940281 * | | 14.37405632 * |
| BNW14 | | 20.1589034 * | | 25.52077188 * |
| BNW15 | | 50.3669741 | | 56.94887208 |
| BNW16 | | 2.70799056 * | | 27.37171929 |
| BNW17 | | 88.2225144 | | 120.1411745 |
| BNW18 | | 92.0338994 | | 82.80931996 |
| BNW19 | | 51.0824709 | | 73.62927124 |
| BNW20 | | 46.8261929 | | 120.655235 |
| BNW21 | | 48.9418364 | | 121.5916615 |
| BNW22 | | 41.3182359 | | 104.3264654 |
| BNW23 | | 85.0676295 | | 132.6608 |
| BNW24 | | 3.93928545 * | | 13.34505396 * |
| BNW25 | | 2.88437681 * | | 13.92786069 * |
| BNW26 | | 94.0659079 | | 136.7564992 |
| BNW27 | | 78.6422701 | | 126.3527217 |
| BNW28 | | 76.7298316 | | 136.975487 |
| BNW29 | | 75.2887485 | | 115.4231371 |
| BNW30 | | 48.3569192 | | 139.9742227 |

### Example 4

Determination of IC50 values, using 7 different inhibitor concentrations at factor 2 intervalls:

| 11β-HSD1 **all values in µM** | | | | | | | |
|---|---|---|---|---|---|---|---|
| | | **BNW 7** | **BNW 13** | **BNW 14** | **BNW 16** | **BNW 24** | **BNW 25** |
| **IC 50** | 1 | 1.95e+0 | 6.66e-1 | 2.75e+0 | 1.49e-1 | 7.33e-1 | 1.47e-1 |
| | 2 | 1.91e+0 | 7.56e-1 | 3.09e+0 | 1.68e-1 | 9.05e-1 | 2.06e-1 |
| | 3 | 2.24e+0 | 6.52e-1 | 2.58e+0 | 1.14e-1 | 7.74e-1 | 1.61e-1 |
| | Mittelwert | 2.03e+0 | 6.91e-1 | 2.81e+0 | 1.44e-1 | 8.04e-1 | 1.72e-1 |
| 11β-HSD2 | Standardabweichung | 0.178522195 | 0.05642599 | 0.25800854 | 0.02724464 | 0.08980411 | 0.03079395 |
| **IC 50** | 1 | did not inhibit | out of range | out of range | out of range | out of range | out of range |
| | 2 | did not inhibit | 2.63e-1 | 2.01e+0 | 4.04e+0 | 1.69e-1 | 5.46e-2 |
| | 3 | did not inhibit | 2.99e-1 | 2.69e+0 | 3.87e+0 | 2.34e-1 | 6.49e-2 |
| | Mittelwert | n.d. | 2.81e-1 | 2.35e+0 | 3.95e+0 | 2.02e-1 | 5.97e-2 |
| | Standardabweichung | n.d. | 0.02520514 | 0.48148793 | 0.11686909 | 0.04659304 | 0.00731635 |

## Claims

1. Use of an 11-β-HSD-type 1 and type 2 inhibitor or a pharmaceutically acceptable salt thereof, for the manufacture of a pharmaceutical agent for the prevention and/or treatment of inflammation-induced and/or immune-mediated loss of bone and/or cartilage, wherein said use is for the prevention and/or treatment of lytic bone metastases, arthritis, juvenile chronic arthritis and/or adjuvant arthritis, infectious diseases, bone loss by HIV, tooth loss, bone marrow inflammation and/or synovial inflammation.

2. The use according to claim 1 for the prevention and/or treatment of inflammation-induced and/or immune-mediated loss of bone and/or cartilage in a mammal.

3. The use according to claim 2, wherein the mammal is a human.

4. The use according to claim 1, wherein said use is for the prevention and/or treatment of periodontitis and/or arthritis selected from the group consisting of osteoarthritis and/or rheumatoid arthritis.

5. The use according to any one of claims 1 to 4, wherein the 11-β-HSD-type 1 and type 2 inhibitor is 18-β-glycyrrhetinic acid or a derivative thereof such as glycyrrhizin or carbenoxolone.

6. The use according to any one of claims 1 to 4, wherein the 11-β-HSD-type 1 and type 2 inhibitor is selected from the group consisting of the following formulas:
| Compound Name | Structure |
|---|---|
| Formula 1 | 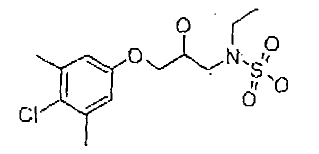 |
| Formula 2 | 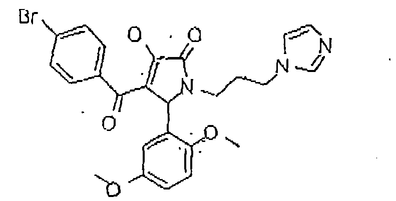 |
| Formula 3 | 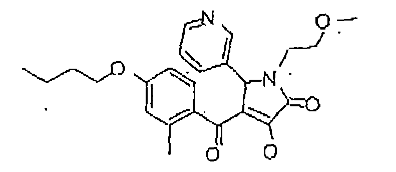 |
| Formula 4 | 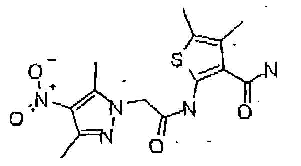 |
| Formula 5 | 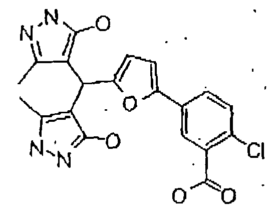 |
| Formula 6 | 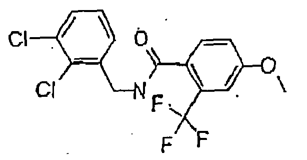 |
| Formula 7 | 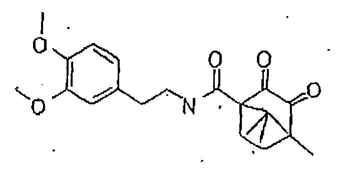 |
| Formula 8 | 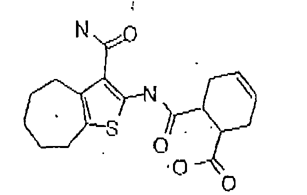 |
| Formula 9 | 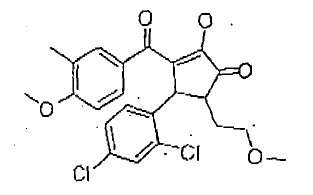 |
| Formula 10 | 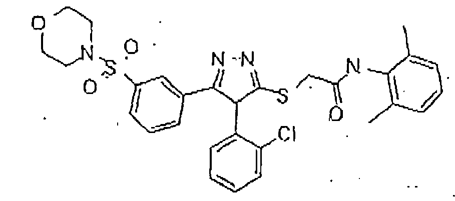 |
| Formula 11 | 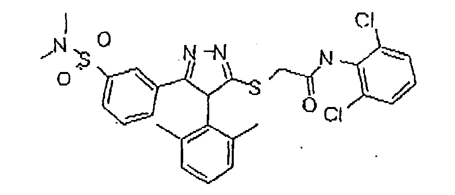 |
| Formula 12 | 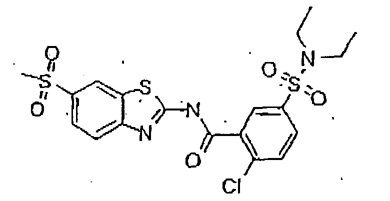 |
| Formula 13 | 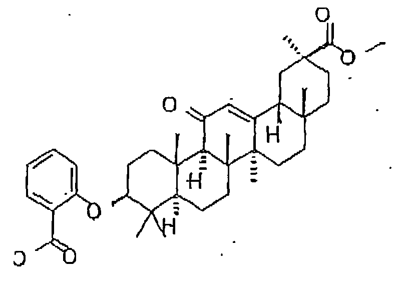 |
| Formula 15 | 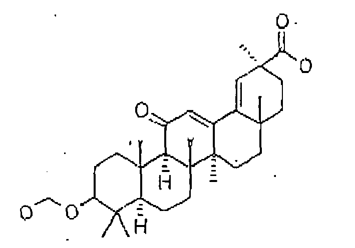 |
| Formula 16 | 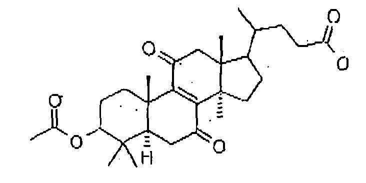 |
| Formula 17 | 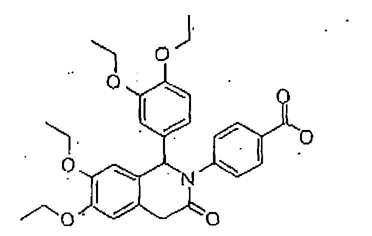 |
| Formula 18 | 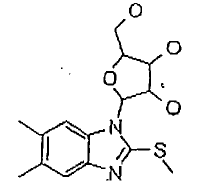 |
| Formula 19 | 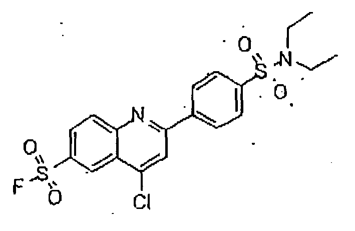 |
| Formula 20 | 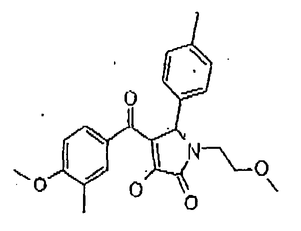 |
| Formula 21 | 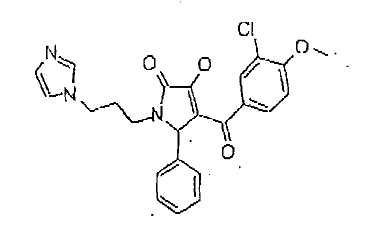 |
| Formula 22 | 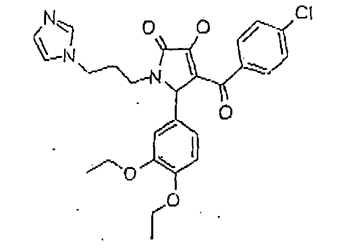 |
| Formula 23 | 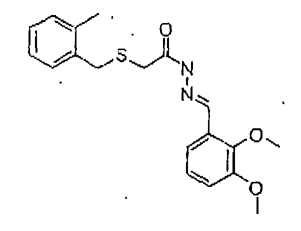 |
| Formula 25 | 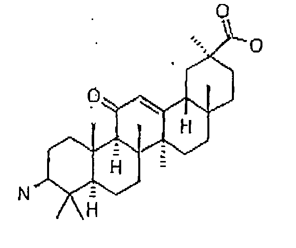 |
| Formula 26 | 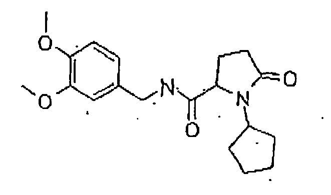 |
| Formula 27 | 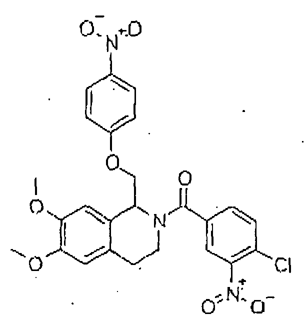 |
| Formula 28 | 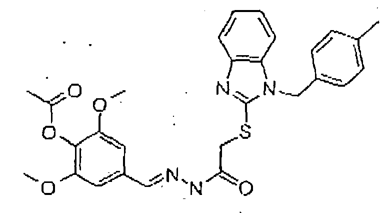 |
| Formula 29 | 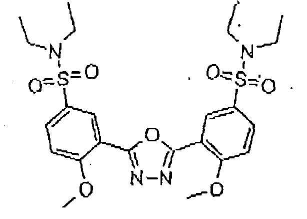 |
| Formula 30 | 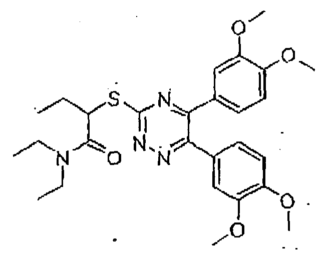 |
| Formula 31 | 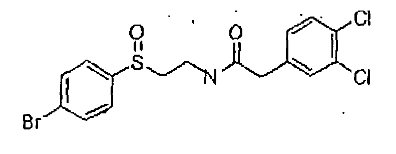 |

7. The use according to claim 6. wherein the 11-p-HSD-type 1 and type 2 inhibitor is selected from the group consisting of the following formulas:
| | |
|---|---|
| Formula 13 | 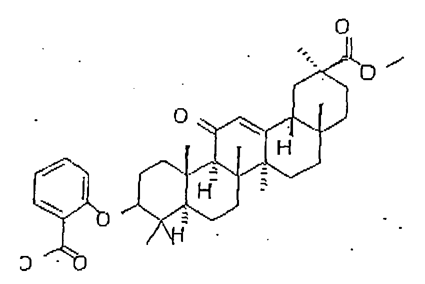 |
| Formula 25 | 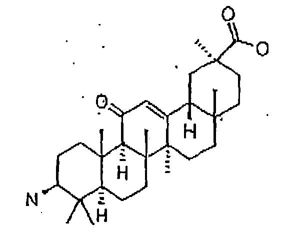 |

8. The use according to claim 1, wherein the 11-β-HSD-type 1 and type 2: inhibitor is:
| | |
|---|---|
| Formula 16 | 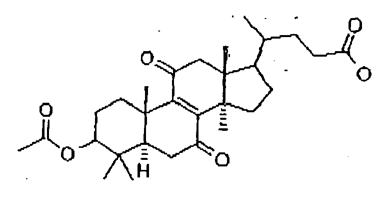 |

9. The use according to claim 6, wherein the 11-β-HSD-type 1 and type 2 inhibitor is:
| | |
|---|---|
| Formula 7 | 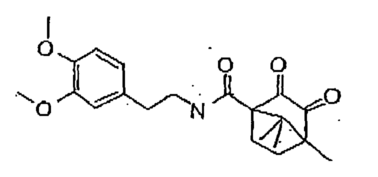 |

10. The use of any one of claims 1 to 9, wherein the pharmaceutical agent comprises at least one 11-β-HSD-type 1 and type 2 inhibitor in combination with at least one active ingredient being effective in the prevention and/or treatment of inflammation-induced and/or immune-mediated loss of bone and/or cartilage.

11. The use according to any one of claims 1 to 10, wherein the pharmaceutical agent is administered in a dose of 5 to 100 mg/kg body weight per day.

12. The use of any one of claims 1 to 11, wherein the Pharmaceutical agent is administered orally, sublingually, intravenously intramuscularly, intraarticularly, intraarterially, intramedullarily, intrathecally, intraventricularly, intraocularly, intracerebrally, intracranially, respiratorally, intratracheally, nasopharyngeally, transdermally, intradermally, subcutaneously, intraperitoneally, intranasally, enterally, topically, via rectal means, via infusion and/or via implant.

13. The use according to claims 12, wherein the pharmaceutical agent is administed orally.

14. The use according to any of claims 1 to 4, wherein the 11-β-HSD-type 1 and type 2 inhibitor is 11-α-OH-progesterone or 11-β-OH-progesterone.

15. Pharmaceutical composition comprising, as an active ingredient, an 11-β-HSD-type 1 and type 2 inhibitor or a salt thereof, wherein said 11-β-HSD-type 1 and type 2 inhibitor is selected from the group consisting of the following formulas 16, 7, 13, and 25:
| | |
|---|---|
| Formula 16 | 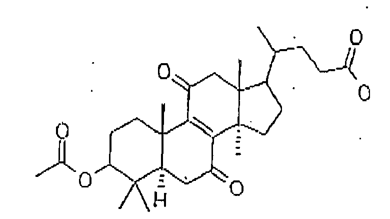 |
| Formula 7 | 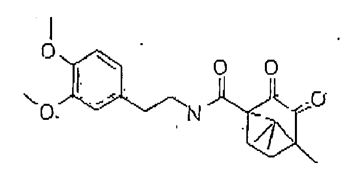 |
| Formula 13 | 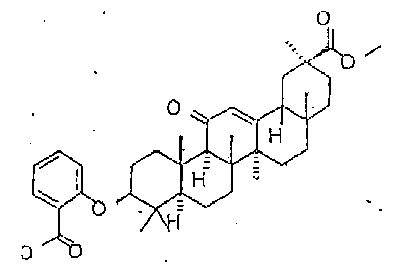 |
| Formula 25 | 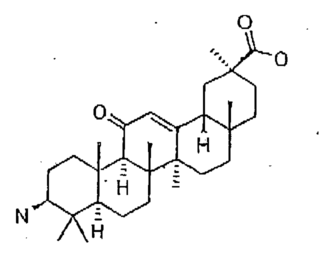 |

16. The use of 18-β-glycyrrhetinic acid for the prevention and/or treatment of inflammation-induced and/or immune-mediated loss of bone and/or cartilage in periodontitis.

17. The use of 18-β-glycyrrhetinic acid for the prevention and/or treatment of inflammation-induced and/or immune-mediated loss of bone and/or cartilage in rheumatoid arthritis.

## Patentansprüche

1. Verwendung eines 11-β-HSD-Typ1- und -Typ2-Inhibitors oder eines pharmazeutisch akzeptablen Salzes davon für die Herstellung eines pharmazeutischen Mittels zur Vorbeugung und/oder Behandlung von entzündungsinduziertem und/oder autoimmunbedingtem Knochenschwund und/oder Knorpelschwund, wobei die Verwendung zur Vorbeugung und/oder Behandlung von lytischen Knochenmetastasen, Arthritis, chronische Arthritis bei Jugendlichen oder Adjuvant-Arthritis, Knochenschwund durch HIV, Zahnschwund, Knochenmarkentzündung und/oder Gelenkentzündung vorgesehen ist.

2. Verwendung nach Anspruch 1 zur Vorbeugung und/oder Behandlung von entzündungsinduziertem und/oder autoimmunbedingtem Knochenschwund und/oder Knorpelschwund bei einem Säugetier.

3. Verwendung nach Anspruch 2, wobei das Säugetier ein Mensch ist.

4. Verwendung nach Anspruch 1, wobei die Verwendung zur Vorbeugung und/oder Behandlung von Parodontitis und/oder Arthritis ausgewählt aus der Gruppe bestehend aus Osteoarthritis und/oder rheumatoider Arthritis vorgesehen ist.

5. Verwendung nach einem der Ansprüche 1 bis 4, wobei der 11-β-HSD-Typ1- und -Typ2-Inhibitor eine 18-β-Glycyrrhetinsäure oder ein Derivat davon, wie Glycyrrhizin oder Carbenoxolon, ist.

6. Verwendung nach einem der Ansprüche 1 bis 4, wobei der 11-β-HSD-Typ1- und -Typ2-Inhibitor ausgewählt ist aus der Gruppe bestehend aus den folgenden Formeln:
| Bezeichnung der Verbindung | Struktur |
|---|---|
| Formel 1 | 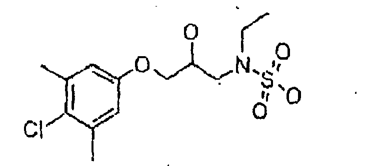 |
| Formel 2 | 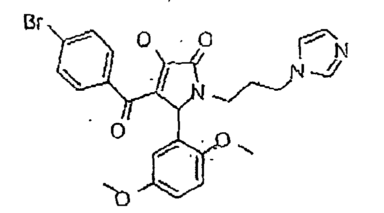 |
| Formel 3 | 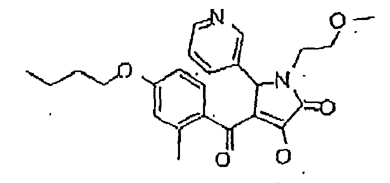 |
| Formel 4 | 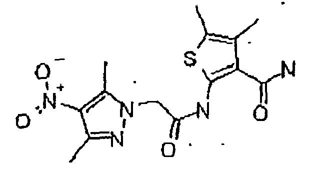 |
| Formel 5 | 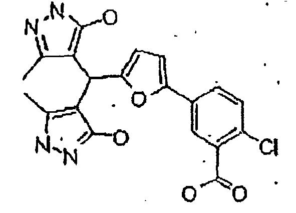 |
| Formel 6 | 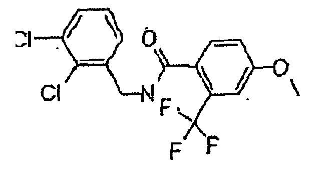 |
| Formel 7 | 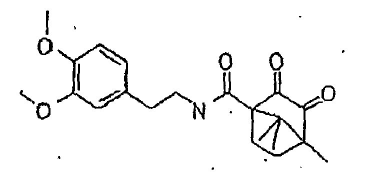 |
| Formel 8 | 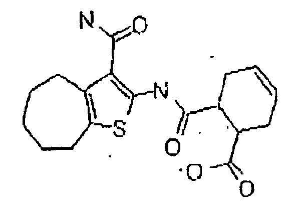 |
| Formel 9 | 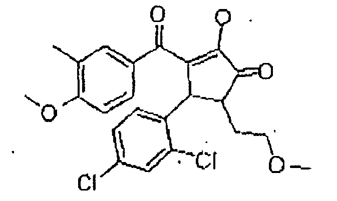 |
| Formel 10 | 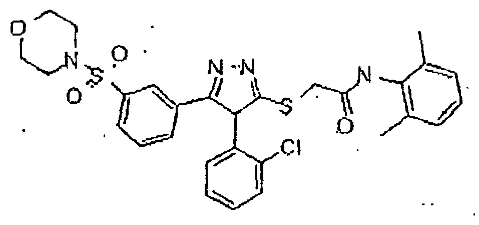 |
| Formel 11 | 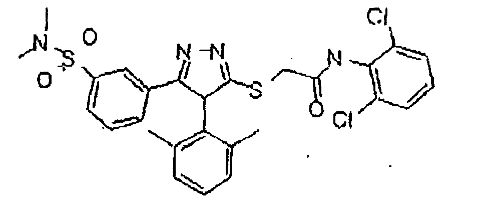 |
| Formel 12 | 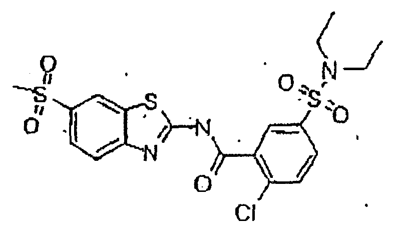 |
| Formel 13 | 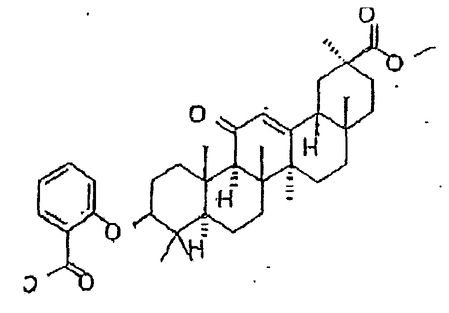 |
| Formel 15 | 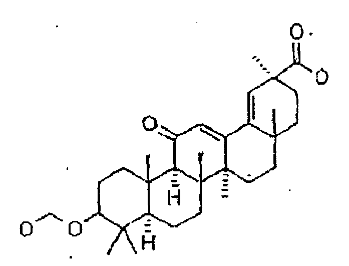 |
| Formel 16 | 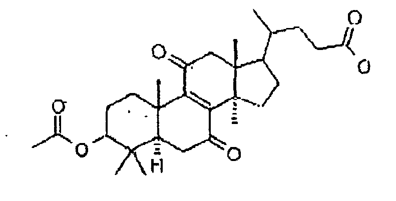 |
| Formel 17 | 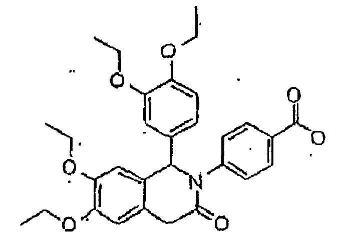 |
| Formel 18 | 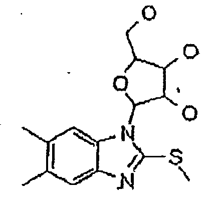 |
| Formel 19 | 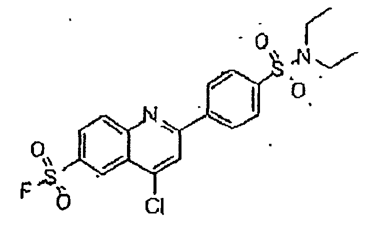 |
| Formel 20 | 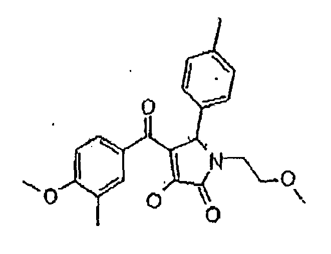 |
| Formel 27 | 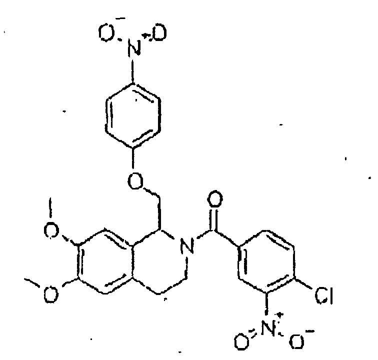 |
| Formel 28 | 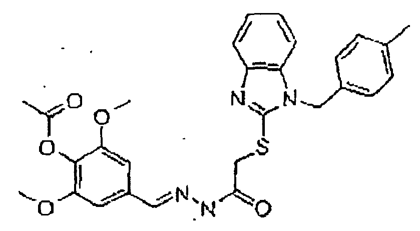 |
| Formel 29 | 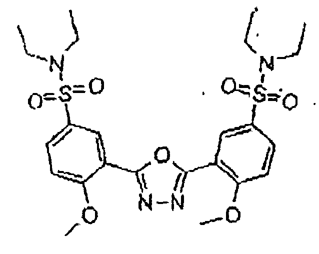 |
| Formel 30 | 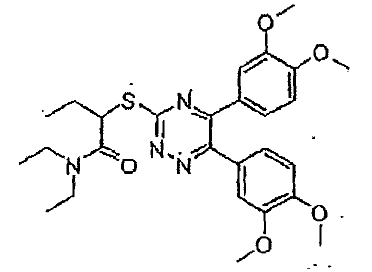 |
| Formel 31 | 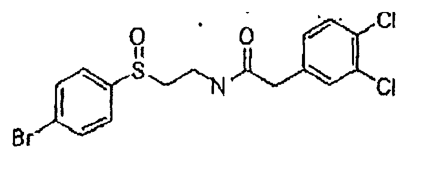 |

7. Verwendung nach Anspruch 6, wobei der 11-β-HSD-Typ1- und -Typ2-Inhibitor ausgewählt ist aus der Gruppe bestehend aus den folgenden Formeln:
| | |
|---|---|
| Formel 13 | 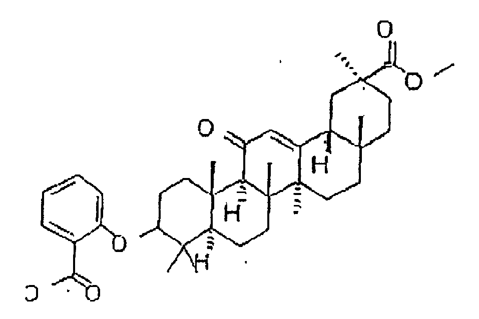 |
| Formel 25 | 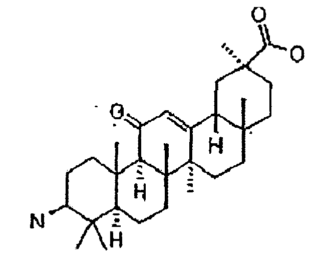 |

8. Verwendung nach Anspruch 1, wobei der 11-β-HSD-Typ1- und -Typ2-Inhibitor
| | |
|---|---|
| Formel 16 | 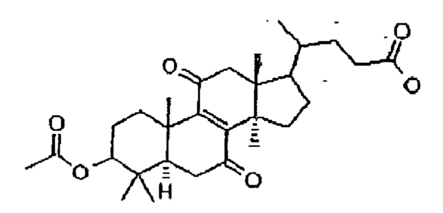 |
ist.

9. Verwendung nach Anspruch 6, wobei der 11-β-HSD-Typ1- und -Typ2-Inhibitor
| | |
|---|---|
| Formel 7 | 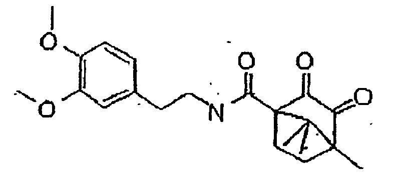 |
ist.

10. Verwendung nach einem der Ansprüche 1 bis 9, wobei das pharmazeutische Mittel zumindest einen 11-β-HSD-Typ1- und -Typ2-Inhibitor in Kombination mit zumindest einem Wirkstoff umfasst, der bei der Vorbeugung und/oder Behandlung von entzündungsinduziertem und/oder autoimmunbedingtem Knochenschwund und/oder Knorpelschwund wirksam ist.

11. Verwendung nach einem der Ansprüche 1 bis 10, wobei das pharmazeutische Mittel täglich in einer Dosis von 5 bis 100 mg/kg Körpergewicht verabreicht wird.

12. Verwendung nach einem der Ansprüche 1 bis 11, wobei das pharmazeutische Mittel oral, unter die Zunge, intravenös, intramuskulär, intraartikulär, intraarteriell, intramedullär, intrathekal, intraventrikulär, intraokular, intrazerebral, intrakraniell, über die Atemwege, intratracheal, nasopharyngeal, transdermal, intradermal, subkutan, intraperitoneal, intranasal, enteral, topisch, rektal, über Infusion und/oder Implantat verabreicht wird.

13. Verwendung nach Anspruch 12, wobei das pharmazeutische Mittel oral verabreicht wird.

14. Verwendung nach einem der Ansprüche 1 bis 4, wobei der 11-β-HSD-Typ1- und -Typ2-Inhibitor ein 11-α-OH-Progesteron oder ein 11-β-OH-Progesteron ist.

15. Pharmazeutische Zusammensetzung, die als Wirkstoff einen 11-β-HSD-Typ1- und -Typ2-Inhibitor oder ein Salz davon umfasst, wobei der 11-β-HSD-Typ1- und -Typ2-Inhibitor ausgewählt ist aus der Gruppe bestehend aus den folgenden Formeln 16, 7, 13 und 25:
| | |
|---|---|
| Formel 16 | 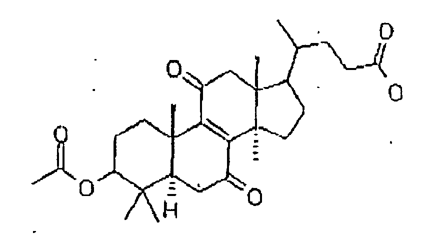 |
| Formel 7 | 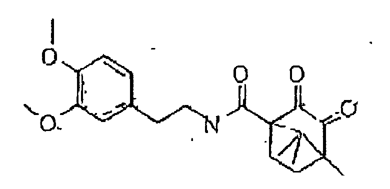 |
| Formel 13 | 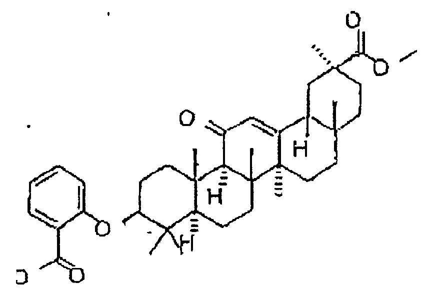 |
| Formel 25 | 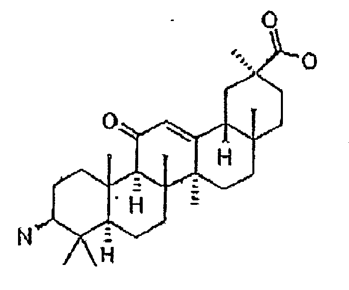 |

16. Verwendung von 18-β-Glycyrrhetinsäure zur Vorbeugung und/oder Behandlung von entzündungsinduziertem und/oder autoimmunbedingtem Knochenschwund und/oder Knorpelschwund bei Parodontitis.

17. Verwendung von 18-β-Glycyrrhetinsäure zur Vorbeugung und/oder Behandlung von entzündungsinduziertem und/oder autoimmunbedingtem Knochenschwund und/oder Knorpelschwund bei rheumatoider Arthritis.

## Revendications

1. Utilisation d'un inhibiteur de type 1 et de type 2 de 11-β-HSD ou d'un sel pharmaceutiquement acceptable de celui-ci pour la production d'un agent pharmaceutique destiné à prévenir et/ou traiter une perte d'os et/ou de cartilage induite par l'inflammation et/ou immuno-médiée, ladite utilisation étant destinée à prévenir et/ou traiter les métastases osseuses lytiques, l'arthrite, l'arthrite chronique juvénile et/ou l'arthrite adjuvante, les maladies infectieuses, la perte osseuse liée au VIH, la perte de dents, l'inflammation de la moelle osseuse et/ou l'inflammation synoviale.

2. Utilisation selon la revendication 1, pour la prévention et/ou le traitement de la perte d'os et/ou de cartilage induite par l'inflammation et/ou immunmédiée chez un mammifère.

3. Utilisation selon la revendication 2, dans laquelle le mammifère est un être humain.

4. Utilisation selon la revendication 1, dans laquelle ladite utilisation est destinée à prévenir et/ou traiter une parodontite et/ou une arthrite dans le groupe comprenant l'arthrose et/ou l'arthrite rhumatoïde.

5. Utilisation selon l'une quelconque des revendications 1 à 4, dans laquelle l'inhibiteur de type 1 et de type 2 de 11-β-HSD est l'acide 18-β-glycyrrhétinique ou un dérivé de celui-ci tel que la glycyrrhizine ou la carbenoxolone.

6. Utilisation selon l'une quelconque des revendications 1 à 4, dans laquelle l'inhibiteur de type 1 et de type 2 de 11-β-HSD est choisi dans le groupe consistant en les formules suivantes :
| Nom du composé | Structure |
|---|---|
| Formule 1 | 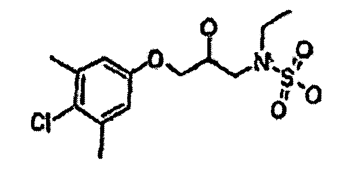 |
| Formule 2 | 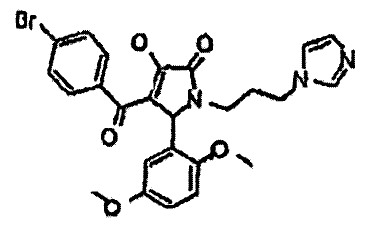 |
| Formule 3 | 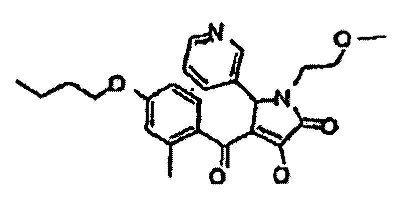 |
| Formule 4 | 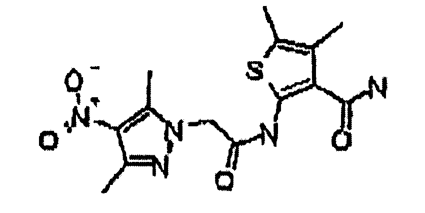 |
| Formule 5 | 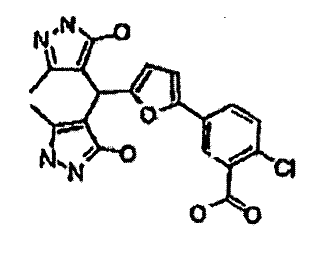 |
| Formule 6 | 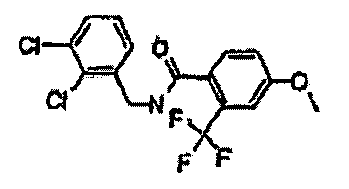 |
| Formule 7 | 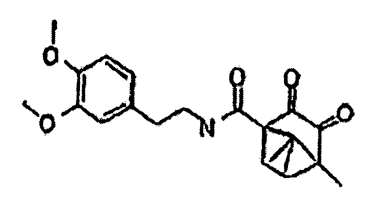 |
| Formule 8 | 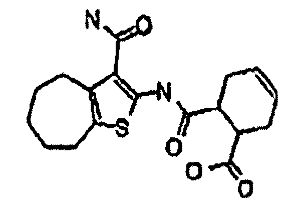 |
| Formule 9 | 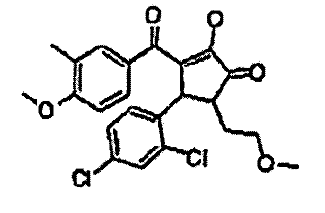 |
| Formule 10 | 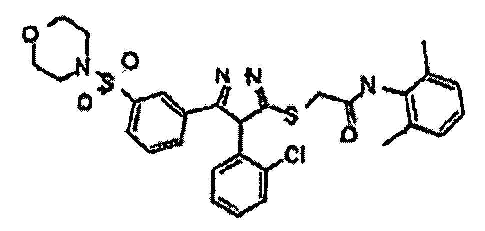 |
| Formule 11 | 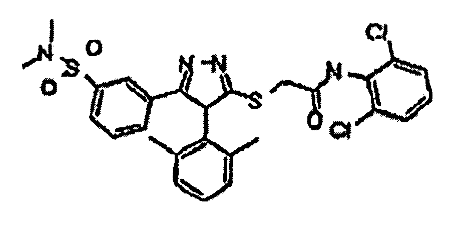 |
| Formule 12 | 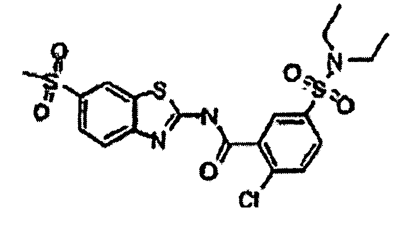 |
| Formule 13 | 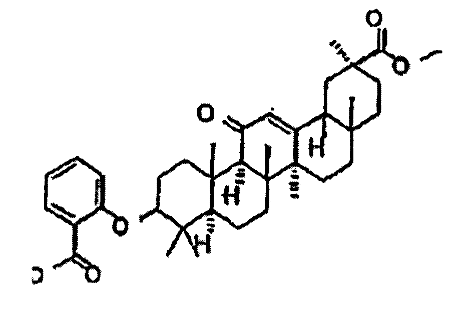 |
| Formule 15 | 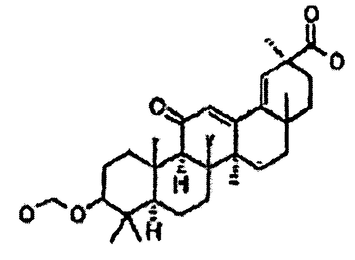 |
| Formule 16 | 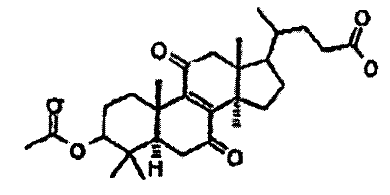 |
| Formule 17 | 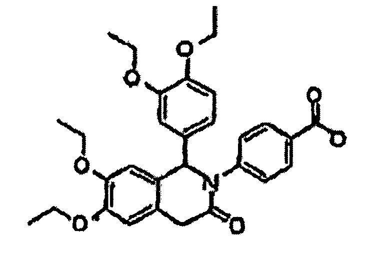 |
| Formule 18 | 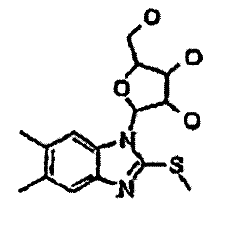 |
| Formule 19 | 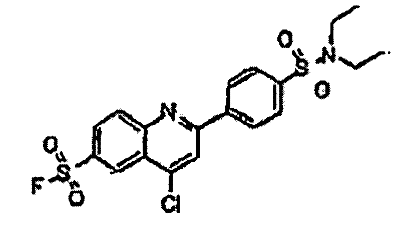 |
| Formule 20 | 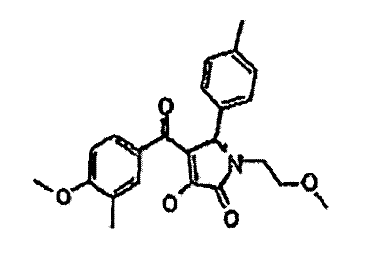 |
| Formule 21 | 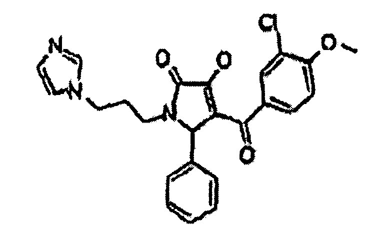 |
| Formule 22 | 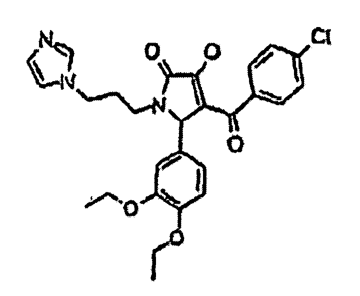 |
| Formule 23 | 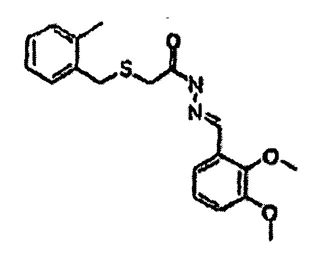 |
| Formule 25 | 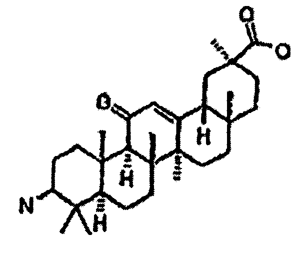 |
| Formule 26 | 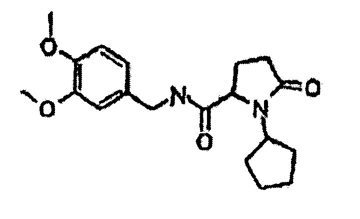 |
| Formule 27 | 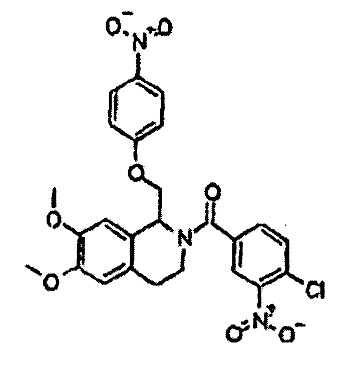 |
| Formule 28 | 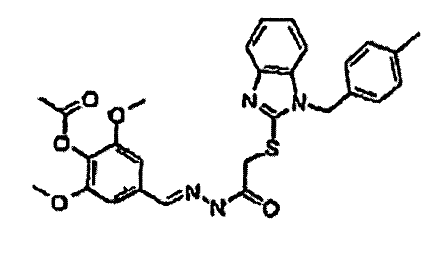 |
| Formule 29 | 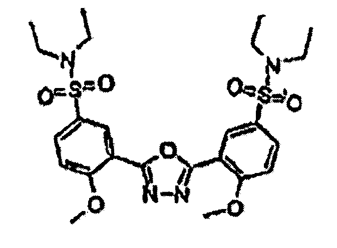 |
| Formule 30 | 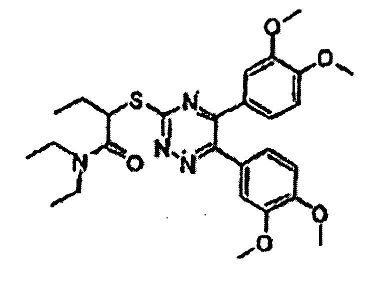 |
| Formule 31 | 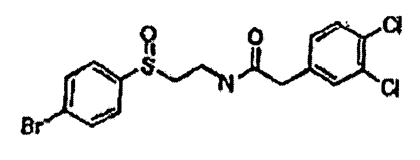 |

7. Utilisation selon la revendication 6, dans laquelle l'inhibiteur de type 1 et de type 2 de 11-β-HSD est choisi dans le groupe consistant en les formules suivantes :
| | | |
|---|---|---|
| Formule 13 | 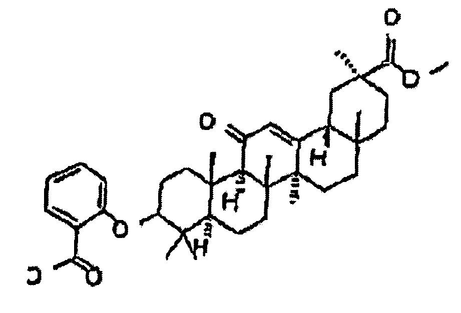 | |
| Formule 25 | 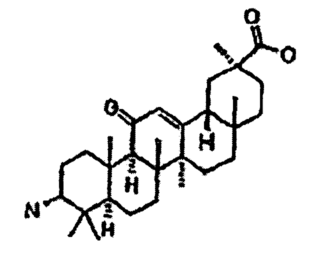 | |

8. Utilisation selon la revendication 1, dans laquelle l'inhibiteur de type 1 et de type 2 de 11-β-HSD est :
| | |
|---|---|
| Formule 16 | 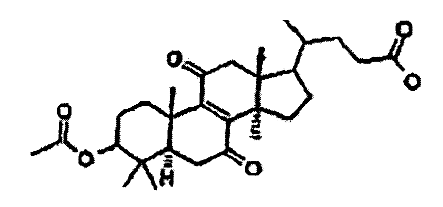 |

9. Utilisation selon la revendication 6, dans laquelle l'inhibiteur de type 1 et de type 2 de 11-β-HSD est :
| | |
|---|---|
| Formule 7 | 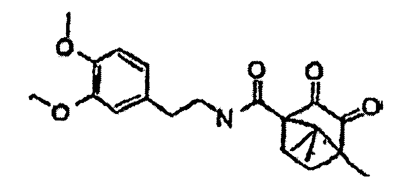 |

10. Utilisation selon l'une quelconque des revendications 1 à 9, dans laquelle l'agent pharmaceutique comprend au moins un inhibiteur de type 1 et de type 2 de 11-β-HSD en combinaison avec au moins un ingrédient actif qui est efficace dans la prévention et/ou le traitement de la perte d'os et/ou de cartilage induite par l'inflammation et/ou immuno-médiée.

11. Utilisation selon l'une quelconque des revendications 1 à 10, dans laquelle l'agent pharmaceutique est administré à une dose de 5 à 100 mg/kg de poids corporel par jour.

12. Utilisation selon l'une quelconque des revendications 1 à 11, dans laquelle l'agent pharmaceutique est administré par voie orale, sublinguale, intraveineuse, intramusculaire, intra-articulaire, intra-artérielle, intra-médulaire, intrathécale, intraventriculaire, intra-oculaire, intracérébrale, intracrânienne, respiratoire, intratrachéale, nasopharyngée, transdermique, intradermique, sous-cutanée, intrapéritonéale, intranasale, entérique, topique, rectale, par perfusion et/ou par implant.

13. Utilisation selon la revendication 12, dans laquelle l'agent pharmaceutique est administré par voie orale.

14. Utilisation selon l'une quelconque des revendications 1 à 4, dans laquelle l'inhibiteur de type 1 et de type 2 de 11-β-HSD est la 11-α-OH-progestérone ou la 11-β-OH-progestérone.

15. Composition pharmaceutique comprenant comme ingrédient actif, un inhibiteur de type 1 et un inhibiteur de type 2 de 11-β-HSD ou un sel de celui-ci, ledit inhibiteur de type 1 et de type de 2 de 11-β-HSD étant choisi dans le groupe consistant en les formules 16, 7, 13 et 25 suivantes :
| | | |
|---|---|---|
| Formule 16 | 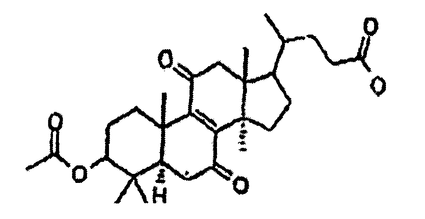 | |
| Formule 7 | 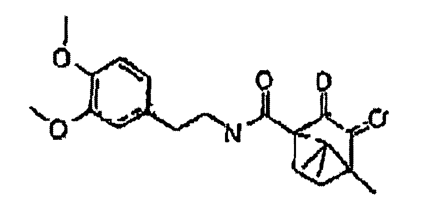 | |
| Formule 13 | 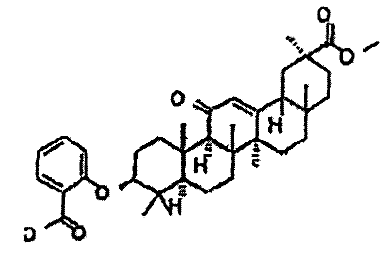 | |
| Formule 25 | 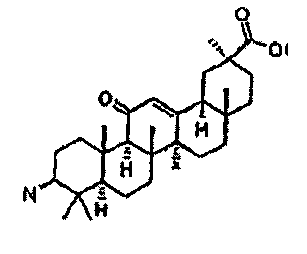 | |

16. Utilisation de l'acide 18-β-glycyrrhétinique pour la prévention et/ou le traitement de la perte d'os et/ou de cartilage induite par l'inflammation et/ou immuno-médiée dans la parodontite.

17. Utilisation de l'acide 18-β-glycyrrhétinique pour la prévention et/ou le traitement de la perte d'os et/ou de cartilage induite par l'inflammation et/ou immuno-médiée dans l'arthrite rhumatoïde.
